# EUROPEAN PATENT SPECIFICATION

(11) **EP 3 880 194 B1**
(45) Date of publication and mention of the grant of the patent: **12.08.2026**
(21) Application number: 19885301.2
(22) Date of filing: 14.11.2019
(51) Int. Cl.: A61K 31/202, A61K 31/232, C07B 59/00, A61P 25/16, A61P 25/28, A61P 25/00, A61P 27/02, A61P 25/18, C07F 9/10, A61K 9/127, A61K 31/685, A61P 39/06, A61P 27/06, C07F 9/113

(54) **DEUTERATED COMPOUNDS FOR USE IN THE TREATMENT OF RETINAL DISORDERS**
DEUTIERTE VERBINDUNGEN ZUR VERWENDUNG BEI DER BEHANDLUNG VON RETINALSTÖRUNGEN
COMPOSÉS DEUTÉRÉS DESTINÉS À ÊTRE UTILISÉS DANS LE TRAITEMENT DES TROUBLES DE LA RÉTINE

(30) Priority: 15.11.2018 US 201862767761 P; 24.07.2019 US 201962878227 P; 03.09.2019 US 201962895438 P
(43) Date of publication of application: 22.09.2021
(73) Proprietor: BioJiva LLC, San Jose, CA 95134 (US)
(72) Inventor: SHCHEPINOV, Mikhail Sergeevich, San Jose, CA 95134 (US)
(74) Representative: Sagittarius IP
(86) International application number: PCT/US2019/061559
(87) International publication number: WO 2020/102596

(56) References cited:
- WO-A1-2011/053870
- WO-A1-2017/091279
- WO-A1-2018/094116
- WO-A1-2019/204582
- WO-A2-2012/148926
- WO-A2-2012/148930
- WO-A2-2015/138773
- LEI PENGXU ET AL: "Mechanisms of Ferroptosis and Relations With Regulated Cell Death: A Review", FRONTIERS IN PHYSIOLOGY, vol. 10, 26 February 2019 (2019-02-26), XP093097980, DOI: 10.3389/fphys.2019.00139
- SMARUN A. V. ET AL: "Site-Specific Deuteration of Polyunsaturated Alkenes", vol. 82, no. 24, 15 December 2017 (2017-12-15), pages 13115 - 13120, XP055838843, ISSN: 0022-3263, Retrieved from the Internet <URL:https://pubs.acs.org/doi/pdf/10.1021/acs.joc.7b02169> DOI: 10.1021/acs.joc.7b02169
- RAEFSKY SOPHIA M. ET AL: "Deuterated polyunsaturated fatty acids reduce brain lipid peroxidation and hippocampal amyloid ?-peptide levels, without discernable behavioral effects in an APP/PS1 mutant transgenic mouse model of Alzheimer's disease", vol. 66, 1 June 2018 (2018-06-01), US, pages 165 - 176, XP055835359, ISSN: 0197-4580, Retrieved from the Internet <URL:https://www.ncbi.nlm.nih.gov/pmc/articles/PMC5924637/pdf/nihms948836.pdf> DOI: 10.1016/j.neurobiolaging.2018.02.024
- FOMICH, MAKSIM A. ET AL.: "Full Library of (Bis-allyl)-deuterated Arachidonic Acids: Synthesis and Analytical Verification", CHEMISTRYSELECT, vol. 1, no. 15, 26 September 2016 (2016-09-26), pages 4758 - 4764, XP055707717, Retrieved from the Internet <URL:https://static1.squarespace.com/static/549af14ae4b004237f7bb71a/t/5813b0dab3db2b2b3a932c1b/1477685509232/Full+Library+of+%28Bis%E2%80%93allyl%29-deuterated+Arachidonic+Acids-Synthesis+and+Analytical+Verification.pdf> DOI: 10.1002/slct.201600955
- NAVRATIL, AARON R. ET AL.: "Lipidomics Reveals Dramatic Physiological Kinetic Isotope Effects during the Enzymatic Oxygenation of Polyunsaturated Fatty Acids Ex Vivo", J. AM. CHEM. SOC., vol. 140, 5 December 2017 (2017-12-05), pages 235 - 243, XP055707719, DOI: 10.1021/jacs.7b09493
- JACQUOT, CYRIL ET AL.: "Isotope Sensitive Branching and Kinetic Isotope Effects in the Reaction of Deuterated Arachidonic Acids with Human 12- and 15-Lipoxygenases", BIOCHEMISTRY, vol. 47, 12 June 2008 (2008-06-12), pages 7295 - 7303, XP055063133, DOI: 10.1021/bi800308q
- LUTHRIA D. L. ET AL.: "Synthesis of ethyl arachidonate-19,19,20,20-d4 and ethyl dihomo-y-linolenate-19,19,20,20-d4", LIPIDS, vol. 28, no. 9, September 1993 (1993-09-01), pages 853 - 856, XP035177682, DOI: 10.1007/BF02536242
- ROSELL, MELISSA ET AL.: "Total Syntheses of Two bis-Allylic-Deuterated DHA Analogues", ASIAN JOURNAL OF ORGANIC CHEMISTRY, vol. 6, no. 3, 13 January 2017 (2017-01-13), pages 322 - 334, XP055707722, Retrieved from the Internet <URL:http://dx.doi.org/10.1002/ajoc.201600565> DOI: 10.1002/ajoc.201600565
- YANG, WAN SEOK ET AL.: "Peroxidation of polyunsaturated fatty acids by lipoxygenases drives ferroptosis", PNAS, vol. 113, no. 34, 9 August 2016 (2016-08-09), pages E4966 - 4975, XP055707724, Retrieved from the Internet <URL:https://doi.org/10.1073/pnas.1603244113> DOI: 10.1073/pnas.1603244113
- FIRSOV ALEXANDER M ET AL.: "Threshold protective effect of deuterated polyunsaturated fatty acids on peroxidation of lipid bilayers", FEBS J, vol. 286, 9 March 2019 (2019-03-09), pages 2099 - 2117, XP055707726
- CHISTYAKOV, DMITRY V. ET AL.: "Deuterated arachidonic acids library for regulation of inflammation and controlled synthesis of eicosanoids: an in vitro study", MOLECULES, vol. 23, no. 12, 15 December 2018 (2018-12-15), pages 3331 - 3341, XP055707732, DOI: 10.3390/molecules23123331
- MAZZA, MARIANNA ET AL.: "Omega-3 fatty acids and antioxidants in neurological and psychiatric diseases: An overview", PROGRESS IN NEURO- PSYCHOPHARMACOLOGY & BIOLOGICAL PSYCHIATRY, vol. 31, 28 August 2006 (2006-08-28), pages 12 - 26, XP005813392, DOI: 10.1016/j.pnpbp.2006.07.010

## Description

### BACKGROUND

### Field

Isotopically modified polyunsaturated fatty acid ("PUFA") compounds, compositions, and methods for reducing lipid autooxidation, ferroptosis, and treating retinal conditions in a subject in need thereof are provided herein.

### Description of the Related Art

Non-enzymatic autoxidation of polyunsaturated fatty acids (PUFAs) damages lipid membranes and generates numerous toxic by-products implicated in neurodegeneration, aging and other pathologies. An imbalance between routine production and detoxification of reactive oxygen species ("ROS") such as peroxides and free radicals can result in oxidative damage to cellular structures and machinery. Under normal conditions, potentially important source of ROSs in aerobic organisms is the leakage of activated oxygen from mitochondria during normal oxidative respiration. Additionally, it is known that macrophages and enzymatic reactions also contribute to the generation of ROSs. Because cells and their internal organelles are lipid membrane-bound, ROSs can readily contact membrane constituents and cause lipid oxidation. Ultimately, such oxidative damage can be relayed to other biomolecules within the cell, such as DNA and proteins, through direct and indirect contact with activated oxygen, oxidized membrane constituents, or other oxidized cellular components. Thus, oxidative damage may propagate throughout a cell given the mobility of internal constituents and the interconnectedness of cellular pathways.

PUFAs are an important sub-class of fatty acids. For homeothermic animals, the two rigorously essential PUFAs are linoleic acid (Lin) and alpha-linolenic acid (Lnn), formerly known as vitamin F. *See* Cunnane, Prog. Lipid Res., 42:544-568 (2003). Lin, by further enzymatic desaturation and elongation, is converted into higher n-6 PUFAs such as arachidonic (Ara; 20:4; n-6) acid; whereas Lnn gives rise to a higher n-3 series, including, but not limited to, eicosapentaenoic acid (EPA; 20:5; n-3) and docosahexaenoic (DHA; 22:6; n-3) acid (Goyens PL. et al. Am. J. Clin. Nutr. 2006; 84:44-53).

PUFAs endow mitochondrial membranes with appropriate fluidity necessary for optimal oxidative phosphorylation performance. PUFAs also play an important role in initiation and propagation of the oxidative stress. PUFAs react with ROS through a chain reaction that amplifies an original event. *See* Sun and Salomon, J. Am. Chem. Soc., 126:5699-5708 (2004). However, non-enzymatic formation of high levels of lipid hydroperoxides is known to result in several detrimental changes. Indeed, Coenzyme Q10 has been linked to increased PUFA toxicity via PUFA autooxidation and the toxicity of the resulting products. *See* Do et al, PNAS USA, 93:7534-7539 (1996). Such oxidized products negatively affect the fluidity and permeability of their membranes; they lead to oxidation of membrane proteins; and they can be converted into a large number of highly reactive carbonyl compounds. *See* Negre-Salvayre et al., Brit. J. Pharmacol., 153:6-20 (2008); *see also,* Esterfbauer et al., Free Rad. Biol. Med., 11:81-128 (1998); and Long and Picklo, Free Rad. Biol. Med., 49:1-8 (2010).

Indeed, lipid autooxidation is increasingly recognized as a major factor in numerous pathological events - neuronal, ocular, vascular and age related. Two features make the lipid autooxidation particularly pernicious: the chain reaction format, and the non-enzymatic nature. The former leads to multiple damage from a single initiating event, while the latter prevents cells from evolving defenses against lipid autooxidation. The lipid autooxidation-induced damage to living systems is multifaceted: compromised membrane fluidity and barrier function are followed by formation of various carbonyl compounds and other highly reactive species which irreversibly cross-link important biomolecules and form mutagenic DNA conjugates. *See, e.g.,* Yin, et al., Chem. Rev. 111:5944-5972 (2011). Lipid bilayer integrity is so vital to neuronal function that 5% of the total energy produced by the body is expended repairing damaged lipids in the brain. *See* Brenna, et al., J. Human Evol. 77:99-106 (2014). To form a membrane, PUFAs (as part of phospholipids) assume a dense, uninterrupted, water-repelling regular formation, which other lipid-soluble molecules can disturb. This may partially explain the inefficiency of antioxidants in inhibiting the lipid autooxidation *in vivo,* established in numerous clinical trials. *See, e.g.,* Kamat, et al., J. Alzheimers Dis. 15:473-493 (2008). The level of antioxidants, such as tocopherols, ascorbate and reduced glutathione, in the oxidative stress-exposed PUFA-rich parts of an organism can be up-regulated by up to 40%. *See* Penn, et al., Exp. Eye Res. 44:779-788

(1987). Under stress, membranes rich in docosahexaenoic acid (DHA) could lose up to 70% of their DHA, which is likely due to both down regulation of the fraction of the most oxidizable PUFA to reduce lipid autooxidation, as well as loss of DHA to oxidation. Similar decrease was observed for PUFAs in general, in animals deficient in vitamin E. *See* Penn, et al., Exp. Eye Res. 44:767-778 (1987); *see also,* Zhang, et al., J. Nutr. 126:2089-2097 (1996). But all in all, tightly controlled delivery mechanisms cannot exceed a certain level of antioxidants in membranes, lest the structural integrity or optimal parameters of the latter be compromised. For example, increasing the level of vitamin E in model bilayers led to decreased fluidity. *See* Urano, et al., Biochem. Biophys. Res. Comm., 150:469-475 (1988). The reported physiological levels of tocopherols relative to fatty acid residues in lipid membranes vary depending on cellular and subcellular membrane types. The ratio of vitamin E to fatty acid residues can be as high as 1:130 in Golgi and lysosomal membranes, but more generally is around 1 tocopherol molecule per 2000 fatty acid residues. *See* Wang, et al., Mol. Membr. Biol. 17:143-156 (2000*); see also,* Halliwell and Gutteridge, Free Radicals in Biology and Medicine; New York: Oxford Univ. Press (2007). But even at this high level of antioxidants, given the stochastic, random nature of ROS generation within the PUFA membranes, and the two-three orders of magnitude difference in the molar ratio of antioxidants and PUFAs, the initiation of the chain reaction of lipid autooxidation cannot be completely inhibited by administering antioxidants, particularly in bilayers rich in long chain PUFAs which are easier to oxidize.

PUFA oxidation is a hallmark of many disease states. For example, oxidative damage in cells of the optic nerve may result in an optic neuropathy such as glaucoma. *See, e.g.,* Izzoti et al., Mutation Research/Reviews in Mutation Research (2006) 612(2), 105-114. Likewise, several studies established oxidative damage as an early event in the pathogenesis of Alzheimer's disease, and such damage can serve as a therapeutic target to slow the progression and/or onset of the disease. *See* Markesbery, Arch. Neurol., 64:954-956 (2007). Similarly, mild cognitive impairment (MCI) can also be characterized by elevated levels of conjugates formed by lipid autooxidation. *See* Butterfield et al., Biochim. Biophys. Acta, 1801:924-929 (2010). WO 2015/138773 discloses the use of deuterated PUFAs for reducing lipid autooxidation and treating glaucoma or optic neuropathy.

Given the major role of lipid autooxidation in cellular damage, and due to fundamental inefficiency of antioxidants in keeping lipid autooxidation in check, other approaches are urgently needed. Thus, there is a need for oxidation resistant PUFAs, that are useful for stabilizing polyunsaturated substances in patients, particularly in patients having oxidation-related disorders or subjected to undesirable oxidative stress.

### SUMMARY

The present invention provides a compound of Formula (I), or a pharmaceutically acceptable salt thereof:

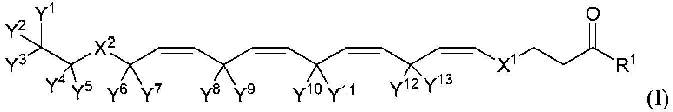

wherein: each of Y¹, Y², Y³, Y⁴, Y⁵, Y⁶, Y⁷, Y⁸, Y⁹, Y¹⁰, Y¹¹, Y¹², Y¹³ ,Y¹⁴, and Y¹⁵ are independently hydrogen and deuterium; X¹ is -C(Y¹⁴Y¹⁵)-CH=CH-, -CH₂-, - CD₂-, or a direct bond; X² is -CH₂CH₂-, -CH₂CD₂-, -CD₂CH₂-, -CD₂CD₂-, or -CH=CH-; R¹ is a substituted or unsubstituted -O-C₁-C₆ alkyl, a substituted or unsubstituted -S-C₁-C₆ alkyl, a substituted or unsubstituted -NH-C₁-C₆ alkyl, -NH₂, -OH, an unsubstituted sphingolipid, or an unsubstituted glyceryl ester; wherein at least two of Y¹, Y², Y³, Y⁴, Y⁵, Y⁶, Y⁷, Y⁸, Y⁹, Y¹⁰, Y¹¹, Y¹², Y¹³ ,Y¹⁴, and Y¹⁵ are independently deuterium; wherein, when each of Y¹-Y⁵ are deuterium, then X¹ is -C(Y¹⁴Y¹⁵)-CH=CH- and at least one of Y⁶-Y¹⁵ is deuterium; or X¹ is -CD₂-; or X² is -CH₂CD₂-, -CD₂CH₂-, or-CD₂CD₂-; and wherein the compound of Formula (I), or a pharmaceutically acceptable salt thereof, comprises from about 1% to about 99%, from about 1% to about 10%, or from about 1% to about 5% of the total amount of fats, fatty acids, and fatty acid esters administered to, or ingested by, the subject, for use in treating or ameliorating a retinal disease or condition caused by lipid autooxidation in a subject in need thereof, wherein the retinal disease or condition is Leber's hereditary optic neuropathy (LHON), macular telangiectasia, or glaucoma. In some further embodiments, the compound of Formula (I), or a pharmaceutically acceptable salt thereof, comprises less than about 5%, less than about 2%, or less than about 1% of the total amount of fats, fatty acids, and fatty acid esters administered to, or ingested by, the subject.

Some non-claimed aspects of the present disclosure provide a method of reducing ferroptosis in a subject in need thereof, comprising administering an effective amount of a compound of Formula (I), or a pharmaceutically acceptable salt thereof, to the subject; wherein the compound of Formula (I), or a pharmaceutically acceptable salt thereof, comprises from about 1% to about 99%, from about 1% to about 10%, or from about 1% to about 5% of the total amount of fats, fatty acids, and fatty acid esters administered to, or ingested by, the subject. In some further embodiments, the compound of Formula (I), or a pharmaceutically acceptable salt thereof, comprises less than about 5%, less than about 2%, or less than about 1% of the total amount of fats, fatty acids, and fatty acid esters administered to, or ingested by, the subject.

Some embodiments provide a compound of formula (I) for treating or ameliorating a retinal condition in a subject in need thereof, comprising administering an effective amount of a compound of Formula (I), or a pharmaceutically acceptable salt thereof, to the subject; wherein the compound of Formula (I), or a pharmaceutically acceptable salt thereof, comprises from about 1% to about 99%, from about 1% to about 10%, or from about 1% to about 5% of the total amount of fats, fatty acids, and fatty acid esters administered to, or ingested by, the subject; and wherein the retinal condition is Leber's hereditary optic neuropathy (LHON), macular telangiectasia, or glaucoma. In some further embodiments, the compound of Formula (I), or a pharmaceutically acceptable salt thereof, comprises less than about 5%, less than about 2%, or less than about 1% of the total amount of fats, fatty acids, and fatty acid esters administered to, or ingested by, the subject.

Some additional embodiments provide a compound of Formula (I) for treating or ameliorating a disease or condition caused by lipid autooxidation or lipid peroxidation in a subject in need thereof, comprising administering an effective amount of a compound of Formula (I), or a pharmaceutically acceptable salt thereof, to the subject. In some embodiments, the compound of Formula (I), or a pharmaceutically acceptable salt thereof, comprises from about 1% to about 99%, from about 1% to about 10%, or from about 1% to about 5% of the total amount of fats, fatty acids, and fatty acid esters administered to, or ingested by, the subject. In some further embodiments, the compound of Formula (I), or a pharmaceutically acceptable salt thereof, comprises less than about 5%, less than about 2%, or less than about 1% of the total amount of fats, fatty acids, and fatty acid esters administered to, or ingested by, the subject. In some embodiments, the compound of Formula (I), or a pharmaceutically acceptable salt thereof is incorporated into the subject's body following administration. In some such embodiments, the subject's body incorporates significant or substantial amount of the compound described herein over the treatment period such that the incorporated compounds in the patient's body is suffient to reduce or prevent lipid autooxidation of the natural (undeuterated) polyunsaturated fatty acid or ester in the subject's body. In some embodiments, the compound (I) also reduces ferroptosis. According to the present invention, the disease or condition is a retinal condition described in claim 1.

Some embodiments provide a liposomal composition comprising from about 1% to about 99% of one or more deuterated compounds in the liposome bilayer, wherein the one or more deuterated compounds are selected from: 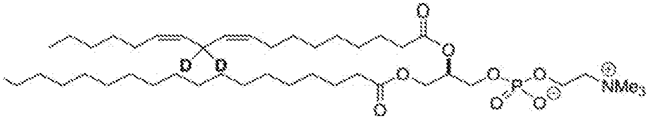 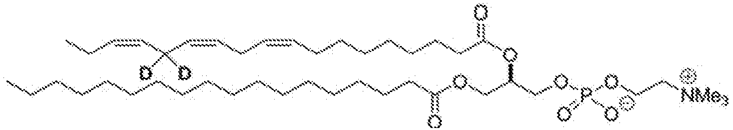 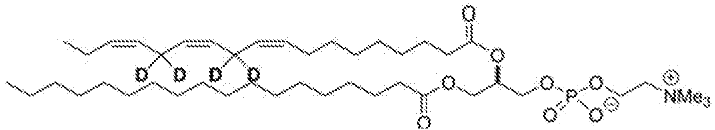 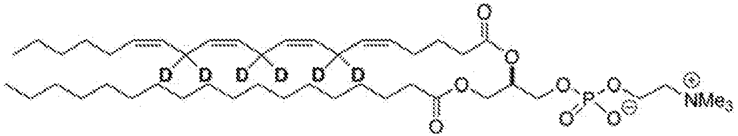 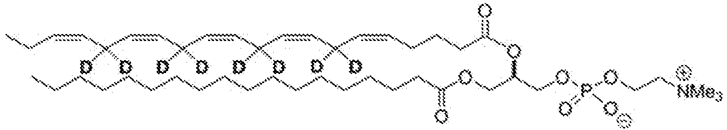 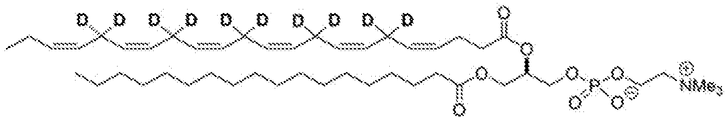 or a salt of any of the foregoing. In some embodiments, the liposomal composition further comprises non-deuterated lipids.

Some embodiments provide a compound of Formula (II), or a pharmaceutically acceptable salt thereof, 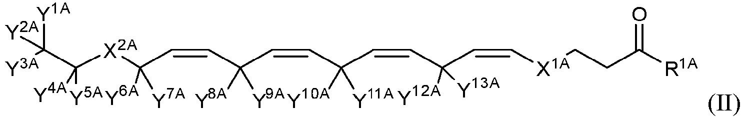 wherein: each of Y^{1A}, Y^{2A}, Y^{3A}, Y^{4A}, Y^{5A}, Y^{6A}, Y^{7A}, Y^{8A}, Y^{9A}, Y^{10A}, Y^{11A}, Y^{12A}, Y1^{3A} ,Y^{14A}, and Y^{15A} are independently hydrogen and deuterium; X^{1A} is -C(Y^{14A}Y^{15A})-CH=CH-, -CH₂-, -CD₂-, or a direct bond; X^{2A} is -CH₂CH₂-, -CH₂CD₂-, -CD₂CH₂-, -CD₂CD₂-, or -CH=CH-; R^{1A} is a substituted or unsubstituted -O-C₁-C₆ alkyl, a substituted or unsubstituted -S-C₁-C₆ alkyl, a substituted or unsubstituted -NH-C₁-C₆ alkyl, -NH₂, -OH, an unsubstituted sphingolipid, an unsubstituted glyceryl ester, or 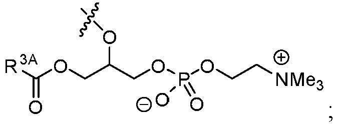 R^{3A} is an unsubstituted C₁-C₂₀ alkyl; wherein at least two of Y^{1A}-Y^{15A} are not hydrogen; and wherein Formula (II) is not selected from: 7,7,10,10,13,13,16,16-D₈-eicosapentaenoic acid, or a salt or ester thereof; 13,13,16,16-D₄-eicosapentaenoic acid, or a salt or ester thereof; 19,19,20,20,20-D₅-eicosapentaenoic acid, or a salt or ester thereof; 21,21,22,22,22-D₅-docosahexaenoic acid, or a salt or ester thereof; 6,6,9,9,12,12,15,15,18,18-D₁₀-docosahexaenoic acid, or a salt or ester thereof; or 7,7,10,10,13,13-D₆-arachadonic acid, or a salt or ester thereof.

### BRIEF DESCRIPTION OF THE DRAWINGS

FIG. 1A depicts a time-dependence of the G(τ) functions of sulforhodamine B (SRB) for liposomes prepared from H-Lin-PC lipids with 10% 1-stearoyl-2-(11,11-D2-linoleyl)-
   sn-glycero-3-phosphatidylcholine (D₂-Lin-PC) measured without Fe²⁺/ascorbate (Fe/Asc) and after the addition of Fe/ascorbate each at 0 min, 10 minutes and 20 minutes respectively.
FIG. 1B depicts a time-dependence of the G(τ) functions of SRB in liposomes prepared from 25% D₂-Lin-PC in H-Lin-PC measured without and with Fe²⁺/ascorbate each at 0 min, 10 minutes and 20 minutes respectively.
FIG. 2A depicts a time course of the extent of liposome leakage for liposomes having 0%, 20%, or 100% D₂-Lin-PC.
FIG. 2B depicts a time course after the addition of Fe²⁺/ascorbate to liposomes having 0%, 20%, or 100% D₂-Lin-PC.
FIG. 3A depicts the difference in α +/- Fe²⁺/ascorbate on the percentage of D₂-Lin-PC in the liposome.
FIG. 3B depicts the dependence of α +/- Fe²⁺/ascorbate at t=10 min for various percentages of D₂-Lin-PC in the liposome.
FIG. 4A depicts a time course of the extent of liposome leakage for liposomes containing arachidonic acid (H-Ara-PC) having various contents of D₆-Ara-PC.
FIG. 4B depicts the dependence of α +/- Fe²⁺/ascorbate for various percentages of D₆-Ara-PC in the liposome.
FIG. 5A depicts a time course of the extent of liposome leakage for liposomes containing H-Lin-PC with varying percentages of D₆-Ara-PC in the H-Lin-PC matrix.
FIG. 5B depicts the dependence of α +/- Fe²⁺/ascorbate for various percentages of D₆-Ara-PC in the liposome.
FIG. 6 depicts the accumulation of diene conjugates at various contents of D₁₀-DHA-PC in the undeuterated H-Lin-PC matrix compared to that for D₈-EPA-PC, D₆-Ara-PC, D₄-Lnn-PC, D₂-Lnn-PC and D₂-Lin-PC.

### DETAILED DESCRIPTION

Note that the references to the methods of treatment by therapy of this description are to be interpreted as references to compounds, pharmaceutical compositions and medicaments of the present invention for use in those methods.

The present disclosure relate to long chain deuterated polyunsaturated fatty acid (PUFA) analogs of Formula (I) and (II) and pharmaceutically acceptable salt thereof. It has been surprisingly discovered that deuterated PUFA analogs with 20, 22 or more carbon atoms chain length have much stronger oxidation preventative effect than the PUFA analogs with short carbon chain length. As such, a lower percentage of the long chain deuterated PUFA analogs may be used for the treatment of various diseases and conditions described herein. In particular, some embodiments provide a PUFA compound, an ester or a derivative thereof, wherein the fatty acid portion of the compound has 20, 22, or more carbon atoms and at least four conjugated double bonds, and wherein each of the bis-allylic position is substituted with a deuterium and the compound has six to fourth deuterium atoms.

### Definitions

Unless otherwise defined, all technical and scientific terms used herein have the same meaning as commonly understood by one of ordinary skill in the art to which these embodiments belong. The terminology used in the description herein is for describing particular embodiments only and is not intended to be limiting of the embodiments. As used in the specification and the appended claims, the singular forms "a," "an," and "the" are intended to include the plural forms as well, unless the context clearly indicates otherwise.

Terms and phrases used in this application, and variations thereof, especially in the appended claims, unless otherwise expressly stated, should be construed as open ended as opposed to limiting. In addition, the term "comprising" is to be interpreted synonymously with the phrases "having at least" or "including at least". When used in the context of a process, the term "comprising" means that the process includes at least the recited steps, but may include additional steps. When used in the context of a compound, composition or device, the term "comprising" means that the compound, composition or device includes at least the recited features or components, but may also include additional features or components.

It is understood that, in any compound described herein having one or more chiral centers, if an absolute stereochemistry is not expressly indicated, then each center may independently be of R-configuration or S-configuration or a mixture thereof. Thus, the compounds provided herein may be enantiomerically pure, enantiomerically enriched, racemic mixture, diastereomerically pure, diastereomerically enriched, or a stereoisomeric mixture. In addition it is understood that, in any compound described herein having one or more double bond(s) generating geometrical isomers that can be defined as E or Z, each double bond may independently be E or Z a mixture thereof.

Likewise, it is understood that, in any compound described, all tautomeric forms are also intended to be included.

It is to be understood that where compounds disclosed herein have unfilled valencies, then the valencies are to be filled with hydrogen atoms.

When hydrogen atom(s) in a PUFA are replaced with deuterium, the PUFA may be referred to, for example, as a D1-PUFA or D₁-PUFA (where one hydrogen is replaced with deuterium), or a D8-PUFA or D₈-PUFA (where eight hydrogen atoms are replaced with deuterium), etc.

As used herein, certain abbreviations are defined as follows:
- Ara: arachidonic acid
- Asc: ascorbate
- D₂-Lin: linoleic acid with full bis-allylic deuteration
- D₄-Lnn: linolenic acid with full bis-allylic deuteration
- D₆-Ara: arachidonic acid with full bis-allylic deuteration
- D₈-EPA: eicosapentaenoic acid with full bis-allylic deuteration
- D₁₀-DHA: docosahexaenoic acid with full bis-allylic deuteration
- D₂-Lin-PC: 1-stearoyl-2-(11,11-D2-linoleyl)-sn-glycero-3-phosphatidylcholine
- D₂-Lnn-PC: 1-stearoyl-2-(14,14-D2-linolenyl)-sn-glycero-3-phosphatidylcholine
- D₄-Lnn-PC: 1-stearoyl-2-(11,11,14,14-D4-linolenyl)-sn-glycero-3-phosphatidylcholine
- D₆-Ara-PC: 1-stearoyl-2-(7,7,10,10,13,13-D6-arachidonoyl)-sn-glycero-3-phosphatidylcholine
- D₈-EPA-PC: 1-stearoyl-2-(7,7,10,10,13,13,16,16-D8-eicosapentaenoyl)-sn-glycero-3-phosphatidylcholine
- D₁₀-DHA-PC: 1-stearoyl-2-(6,6,9,9,12,12,15,15,18,18-D10-docosahexaenoyl)-sn-glycero-3-phosphatidylcholine
- DHA: docosahexaenoic acid
- D-PUFA: PUFA having bis-allylic hydrogen atoms replaced with deuterium atoms
- EPA: eicosapentaenoic acid
- EtOAc: ethyl acetate
- FCS: fluorescence correlation spectroscopy
- H-Ara-PC: 1-stearoyl-2-arachidonoyl-sn-glycero-3-phosphatidylcholine
- H-Lin-PC: 1-stearoyl-2-linoleyl-sn-glycero-3-phosphatidylcholine
- H-LPC: hydrogenated lysophosphatidylcholine
- HNE: 4-hydroxy-2-nonenal
- KIE: kinetic isotope effect
- Lin: linoleic acid
- Lnn: linolenic acid
- LPO: lipid peroxidation
- MDA: malondialdehyde
- MsCl: methanesulfonyl chloride
- OCR: oxygen consumption rate;
- Ole: oleic acid
- PUFA: polyunsaturated fatty acid
- ROS: reactive oxygen species
- SRB: sulforhodamine B

As used herein, "Cₐ to C_{b}" in which "a" and "b" are integers refer to the number of carbon atoms in an alkyl, alkenyl or alkynyl group, or the number of carbon atoms in the ring of a cycloalkyl, aryl, heteroaryl or heterocyclyl group. That is, the alkyl, alkenyl, alkynyl, ring of the cycloalkyl, ring of the aryl, ring of the heteroaryl or ring of the heterocyclyl can contain from "a" to "b", inclusive, carbon atoms. Thus, for example, a "C₁ to C₄ alkyl" group or a "C₁-C₄ alkyl" group refers to all alkyl groups having from 1 to 4 carbons, that is, CH₃-, CH₃CH₂-, CH₃CH₂CH₂-, (CH₃)₂CH-, CH₃CH₂CH₂CH₂-, CH₃CH₂CH(CH₃)-and (CH₃)₃C-. Likewise, for example, cycloalkyl group may contain from "a" to "b", inclusive, total atoms, such as a C₃-C₈ cycloalkyl group, 3 to 8 carbon atoms in the ring(s). If no "a" and "b" are designated with regard to an alkyl, cycloalkyl, or cycloalkenyl, the broadest range described in these definitions is to be assumed.

As used herein, "alkyl" refers to a monovalent straight or branched hydrocarbon chain that comprises a fully saturated (no double or triple bonds) hydrocarbon group. The alkyl group may have 1 to 20 carbon atoms (whenever it appears herein, a numerical range such as "1 to 20" refers to each integer in the given range; e.g., "1 to 20 carbon atoms" means that the alkyl group may consist of 1 carbon atom, 2 carbon atoms, 3 carbon atoms, etc., up to and including 20 carbon atoms, although the present definition also covers the occurrence of the term "alkyl" where no numerical range is designated). The alkyl group may also be a medium size alkyl having 1 to 10 carbon atoms. The alkyl group could also be a lower alkyl having 1 to 6 carbon atoms. The alkyl group of the compounds may be designated as "C₁-C₄ alkyl" or similar designations. By way of example only, "C₁-C₄ alkyl" indicates that there are one to four carbon atoms in the alkyl chain, *i.e.,* the alkyl chain is selected from methyl, ethyl, propyl, isopropyl, n-butyl, isobutyl, sec-butyl, and t-butyl. Typical alkyl groups include, but are in no way limited to, methyl, ethyl, propyl, isopropyl, butyl, isobutyl, tertiary butyl, pentyl, and hexyl.

The term "alkenyl" used herein refers to a monovalent straight or branched chain radical of from 2 to 20 carbon atoms containing a carbon double bond(s) including, but not limited to, 1-propenyl, 2-propenyl, 2-methyl-1-propenyl, 1-butenyl, 2-butenyl and the like.

As used herein, "cycloalkyl" refers to a completely saturated (no double or triple bonds) mono- or multi- cyclic hydrocarbon ring system. When composed of two or more rings, the rings may be joined together in a fused, bridged or spiro fashion. As used herein, the term "fused" refers to two rings which have two atoms and one bond in common. As used herein, the term "bridged cycloalkyl" refers to compounds wherein the cycloalkyl contains a linkage of one or more atoms connecting non-adjacent atoms. As used herein, the term "spiro" refers to two rings which have one atom in common and the two rings are not linked by a bridge. Cycloalkyl groups can contain 3 to 30 atoms in the ring(s), 3 to 20 atoms in the ring(s), 3 to 10 atoms in the ring(s), 3 to 8 atoms in the ring(s) or 3 to 6 atoms in the ring(s). Examples of mono-cycloalkyl groups include, but are in no way limited to, cyclopropyl, cyclobutyl, cyclopentyl, cyclohexyl, cycloheptyl and cyclooctyl.

As used herein, "aryl" refers to a carbocyclic (all carbon) monocyclic or multicyclic aromatic ring system (including fused ring systems where two carbocyclic rings share a chemical bond) that has a fully delocalized pi-electron system throughout all the rings. The number of carbon atoms in an aryl group can vary. For example, the aryl group can be a C₆-C₁₄ aryl group, a C₆-C₁₀ aryl group or a C₆ aryl group. Examples of aryl groups include, but are not limited to, benzene, naphthalene and azulene.

As used herein, "heteroaryl" refers to a monocyclic or multicyclic aromatic ring system (a ring system with fully delocalized pi-electron system) that contain(s) one or more heteroatoms (for example, 1, 2 or 3 heteroatoms), that is, an element other than carbon, including but not limited to, nitrogen, oxygen and sulfur. The number of atoms in the ring(s) of a heteroaryl group can vary. For example, the heteroaryl group can contain 4 to 14 atoms in the ring(s), 5 to 10 atoms in the ring(s) or 5 to 6 atoms in the ring(s), such as nine carbon atoms and one heteroatom; eight carbon atoms and two heteroatoms; seven carbon atoms and three heteroatoms; eight carbon atoms and one heteroatom; seven carbon atoms and two heteroatoms; six carbon atoms and three heteroatoms; five carbon atoms and four heteroatoms; five carbon atoms and one heteroatom; four carbon atoms and two heteroatoms; three carbon atoms and three heteroatoms; four carbon atoms and one heteroatom; three carbon atoms and two heteroatoms; or two carbon atoms and three heteroatoms. Furthermore, the term "heteroaryl" includes fused ring systems where two rings, such as at least one aryl ring and at least one heteroaryl ring or at least two heteroaryl rings, share at least one chemical bond. Examples of heteroaryl rings include, but are not limited to, furan, furazan, thiophene, benzothiophene, phthalazine, pyrrole, oxazole, benzoxazole, 1,2,3-oxadiazole, 1,2,4-oxadiazole, thiazole, 1,2,3-thiadiazole, 1,2,4-thiadiazole, benzothiazole, imidazole, benzimidazole, indole, indazole, pyrazole, benzopyrazole, isoxazole, benzoisoxazole, isothiazole, triazole, benzotriazole, thiadiazole, tetrazole, pyridine, pyridazine, pyrimidine, pyrazine, purine, pteridine, quinoline, isoquinoline, quinazoline, quinoxaline, cinnoline and triazine.

As used herein, "heterocyclyl" refers to three-, four-, five-, six-, seven-,eight-, nine-, ten-, up to 18-membered monocyclic, bicyclic and tricyclic ring system wherein carbon atoms together with from 1 to 5 heteroatoms constitute said ring system. A heterocycle may optionally contain one or more unsaturated bonds situated in such a way, however, that a fully delocalized pi-electron system does not occur throughout all the rings. The heteroatom(s) is an element other than carbon including, but not limited to, oxygen, sulfur and nitrogen. A heterocycle may further contain one or more carbonyl or thiocarbonyl functionalities, so as to make the definition include oxo-systems and thio-systems such as lactams, lactones, cyclic imides, cyclic thioimides and cyclic carbamates. When composed of two or more rings, the rings may be joined together in a fused, bridged or spiro fashion. As used herein, the term "fused" refers to two rings which have two atoms and one bond in common. As used herein, the term "bridged heterocyclyl" or "bridged heteroalicyclyl" refers to compounds wherein the heterocyclyl contains a linkage of one or more atoms connecting non-adjacent atoms. As used herein, the term "spiro" refers to two rings which have one atom in common and the two rings are not linked by a bridge. Heterocyclyl groups can contain 3 to 30 atoms in the ring(s), 3 to 20 atoms in the ring(s), 3 to 10 atoms in the ring(s), 3 to 8 atoms in the ring(s) or 3 to 6 atoms in the ring(s). For example, five carbon atoms and one heteroatom; four carbon atoms and two heteroatoms; three carbon atoms and three heteroatoms; four carbon atoms and one heteroatom; three carbon atoms and two heteroatoms; two carbon atoms and three heteroatoms; one carbon atom and four heteroatoms; three carbon atoms and one heteroatom; or two carbon atoms and one heteroatom. Additionally, any nitrogens in a heteroalicyclic may be quaternized. Examples of heterocyclyl groups include but are not limited to, 1,3-dioxin, 1,3-dioxane, 1,4-dioxane, 1,2-dioxolane, 1,3-dioxolane, 1,4-dioxolane, 1,3-oxathiane, 1,4-oxathiin, 1,3-oxathiolane, 1,3-dithiole, 1,3-dithiolane, 1,4-oxathiane, tetrahydro-1,4-thiazine, 2H-1,2-oxazine, maleimide, succinimide, barbituric acid, thiobarbituric acid, dioxopiperazine, hydantoin, dihydrouracil, trioxane, hexahydro-1,3,5-triazine, imidazoline, imidazolidine, isoxazoline, isoxazolidine, oxazoline, oxazolidine, oxazolidinone, thiazoline, thiazolidine, morpholine, oxirane, piperidine N-Oxide, piperidine, piperazine, pyrrolidine, azepane, pyrrolidone, pyrrolidione, 4-piperidone, pyrazoline, pyrazolidine, 2-oxopyrrolidine, tetrahydropyran, 4H-pyran, tetrahydrothiopyran, thiamorpholine, thiamorpholine sulfoxide, thiamorpholine sulfone and their benzo-fused analogs (e.g., benzimidazolidinone, tetrahydroquinoline and/or 3,4-methylenedioxyphenyl).

As used herein, the term "hydroxy" refers to a -OH group.

As used herein, "alkoxy" refers to the Formula -OR wherein R is an alkyl, an alkenyl, a cycloalkyl, aryl, heteroaryl, or heterocyclyl, as defined herein. A non-limiting list of alkoxys are methoxy, ethoxy, n-propoxy, 1-methylethoxy (iso-propoxy), n-butoxy, iso-butoxy, sec-butoxy, tert-butoxy, phenoxy and benzoxy.

A "cyano" group refers to a "-CN" group.

The term "halogen atom" or "halogen" as used herein, means any one of the radio-stable atoms of column 7 of the Periodic Table of the Elements, such as, fluorine, chlorine, bromine and iodine.

A "nitro" group refers to an "-NO₂" group.

As used herein, "haloalkyl" refers to an alkyl group in which one or more of the hydrogen atoms are replaced by a halogen (e.g., mono-haloalkyl, di-haloalkyl, tri-haloalkyl and polyhaloalkyl). Such groups include but are not limited to, chloromethyl, fluoromethyl, difluoromethyl, trifluoromethyl, 1-chloro-2-fluoromethyl, 2-fluoroisobutyl and pentafluoroethyl.

As used herein, "haloalkoxy" refers to an alkoxy group in which one or more of the hydrogen atoms are replaced by a halogen (e.g., mono-haloalkoxy, di- haloalkoxy and tri-haloalkoxy). Such groups include but are not limited to, chloromethoxy, fluoromethoxy, difluoromethoxy, trifluoromethoxy, 1-chloro-2-fluoromethoxy and 2-fluoroisobutoxy.

The term "amino" as used herein refers to a -NH₂ group.

Whenever a group is described as being "unsubstituted or substituted" if substituted, the substituent(s) may be selected from one or more the indicated substituents. If no substituents are indicated, it is meant that the indicated "substituted" group may be substituted with one or more group(s) individually and independently selected from alkyl, alkenyl, cycloalkyl, aryl, heteroaryl, heterocyclyl, hydroxy, alkoxy, cyano, halogen, nitro, haloalkyl, haloalkoxy, and amino.

As used herein, the term "glyceryl ester" refers to an ester of glycerol having the structure: 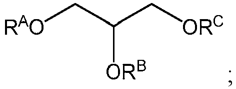 where R^{A}, R^{B}, and R^{C} are independently hydrogen, a compound of Formula (I), -(C=O)-C₁-C₂₀ alkyl, or -(C=O)-C₂-C₂₀ alkenyl, with at least one of R^{A}, R^{B}, and R^{C} being a compound of Formula (I). When two of R^{A}, R^{B}, and R^{C} are hydrogen, the glyceryl ester is a mono-glyceryl ester. When one of R^{A}, R^{B}, and R^{C} are hydrogen, the glyceryl ester is a di-glyceryl ester. When none of R^{A}, R^{B}, and R^{C} are hydrogen, the glyceryl ester is a triglyceryl ester. Examples of glyceryl esters that can be appended to compounds of Formula (I) include, but are not limited to phosphatidylethanolamines, phosphatidylcholines, phosphatidylserines, phosphoinositides, ceramides, and sphingomyelins.

As used herein, the terms "treat," "treating," "treatment," "therapeutic," and "therapy" do not necessarily mean total cure or abolition of the disease or condition. Any alleviation of any undesired signs or symptoms of a disease or condition, to any extent can be considered treatment and/or therapy. Furthermore, treatment may include acts that may worsen the subject's overall feeling of well-being or appearance.

The term "effective amount" is used to indicate an amount of an active compound, or pharmaceutical agent, that elicits the biological or medicinal response indicated. For example, an effective amount of compound can be the amount needed to prevent, alleviate or ameliorate symptoms of a disease or condition, improve the quality of life of and/or prolong the survival of, the subject being treated. This response may occur in a tissue, system, animal or human and includes alleviation of the signs or symptoms of the condition being treated. Determination of an effective amount is well within the capability of those skilled in the art, in view of the disclosure provided herein. The effective amount of the compounds disclosed herein required as a dose will depend on the route of administration, the type of animal, including human, being treated, and the physical characteristics of the specific animal under consideration. The dose can be tailored to achieve a desired effect, but will depend on such factors as weight, diet, concurrent medication and other factors which those skilled in the medical arts will recognize.

As used herein, the term "ferroptosis" refers to an iron-dependent programmed cell death pathway caused by failure of antioxidant defenses, resulting in unchecked lipid autooxidation and cell death.

As used herein, the terms "bis-allylic" or "bis-allylic position" generally refers to the position of a polyunsaturated substance, e.g., a polyunsaturated fatty acid or mimetic thereof, that corresponds to the methylene groups of a 1,4-diene system.

As used herein, the terms "pro-bis-allylic" or "pro-bis-allylic position" refers to the methylene group that becomes the bis-allylic position upon enzymatic desaturation.

As used herein, the term "PUFA" refers to a polyunsaturated fatty acid. Unless otherwise specified, the term "PUFA" also includes both salts of the fatty acids and esters of the fatty acids. Accordingly, the term "PUFA" includes pharmaceutically acceptable polyunsaturated fatty acid salts, and pharmaceutically acceptable polyunsaturated fatty acid esters.

As used herein, the term "concurrently" means at substantially the same time.

As used herein, the term "autooxidation" refers to autocatalytic oxidation, e.g. autocatalytic lipid peroxidation.

Unless otherwise stated, when a position is designated specifically as "H" or "hydrogen", the position is understood to have hydrogen at its natural abundance isotopic composition. Unless otherwise stated, when a position is designated specifically as "D" or "deuterium", the position has deuterium at an abundance that is at least 3206 times of the natural abundance of deuterium, which is 0.0156% (i.e., at least 50% deuterium incorporation). More specifically, the position may have deuterium at an abundance that is at least 3500 times (54.6% deuterium incorporation), 4000 times (62.4% deuterium incorporation), 4500 times (70.2% deuterium incorporation), 5000 times (78% deuterium incorporation), 5500 times (85.8% deuterium incorporation), 6000 times (93.6% deuterium incorporation), 6090 times (95% deuterium incorporation), 6250 times (97.5% deuterium incorporation), 6346 times (99% deuterium incorporation), or 6378 times (99.5% deuterium incorporation) of the natural abundance of deuterium.

The term "isotopologue" refers to a molecule that differ only in its isotopic composition. It has the same chemical formula and bonding arrangement of atoms, but at least one atom has a different number of neutrons than the parent compound.

The term "compound," when referring to a deuterated compound of Formula (I) or (II) as described herein, refers to a collection of molecules having an identical chemical structure, except that there may be isotopic variation among the constituent atoms of the molecules. One of ordinary skill in the art understands that a compound represented by a particular chemical structure containing indicated deuterium atoms, will also contain lesser amounts of isotopologues having hydrogen atoms at one or more of the designated deuterium positions in that structure. The relative amount of such isotopologues in total will be less than 49.9% of the compound. In other embodiments, the relative amount of such isotopologues in toto will be less than 40%, less than 30%, less than 20%, less than 15%, less than 10%, less than 5%, less than 2%, less than 1%, less than 0.5%, or less than 0.1% of the deuterated compounds described herein.

### Compounds

One skilled in the art will readily appreciate that when one of the two hydrogens of a methylene group is replaced with a deuterium atom, the resultant compound may possess a stereocenter. In some embodiments, the compounds of Formula (I), and pharmaceutically acceptable salts thereof, and compounds of Formula (II), and pharmaceutically acceptable salts thereof, are racemic. In other embodiments, the compounds of Formula (I), and pharmaceutically acceptable salts thereof, and compounds of Formula (II), and pharmaceutically acceptable salts thereof, are enantiomerically pure. In additional embodiments, the compounds of Formula (I), and pharmaceutically acceptable salts thereof, and compounds of Formula (II), and pharmaceutically acceptable salts thereof, are diastereomerically pure. In some embodiments, the compounds of Formula (I), and pharmaceutically acceptable salts thereof, and compounds of Formula (II), and pharmaceutically acceptable salts thereof, have enantiomeric excesses and/or diastereomeric excesses of about 5%, 10%, 15%, 20%, 25%, 30%, 35%, 40%, 45%, 50%, 65%, 60%, 65%, 70%, 75%, 80%, 85%, 90%, 95%, or 100%, or a range bounded by any two of the aforementioned numbers. In other embodiments, the enantiomeric excesses and/or diastereomeric excesses is at least 5%, 10%, 15%, 20%, 25%, 30%, 35%, 40%, 45%, 50%, 65%, 60%, 65%, 70%, 75%, 80%, 85%, 90%, 95%, or 100%, or a range bounded by any two of the aforementioned numbers.

Some embodiments provide a compound of Formula (II), or a pharmaceutically acceptable salt thereof:

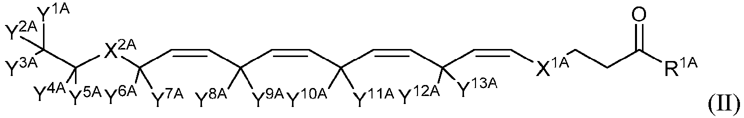

In some embodiments, each of Y^{1A}, Y^{2A}, Y^{3A}, Y^{4A}, Y^{5A}, Y^{6A}, Y^{7A}, Y^{8A}, Y^{9A}, Y^{10A}, Y^{11A}, Y^{12A}, Y^{13A} ,Y^{14A}, and Y^{15A} are independently hydrogen and deuterium. A variety of combinations of hydrogen and deuterium substitution between Y^{1A} and Y^{13A} is contemplated herein. For example, in some instances, each of Y^{1A}, Y^{2A}, Y^{3A}, Y^{4A}, Y^{5A}, Y^{6A}, Y^{7A}, Y^{8A}, Y^{9A}, Y^{10A}, Y^{11A}, Y^{12A}, Y^{13A} ,Y^{14A}, and Y^{15A} are deuterium. In other instances, each of Y^{1A}, Y^{2A}, Y^{3A}, Y^{4A}, and Y^{5A} are hydrogen; two of Y^{6A}, Y^{7A}, Y^{8A}, Y^{9A}, Y^{10A}, Y^{11A}, Y^{12A}, Y^{13A} ,Y^{14A}, and Y^{15A} are deuterium; and the remainder of Y^{6A}, Y^{7A}, Y^{8A}, Y^{9A}, Y^{10A}, Y^{11A}, Y^{12A}, Y^{13A} ,Y^{14A}, and Y^{15A} are hydrogen. In still other instances, each of Y^{1A}, Y^{2A}, Y^{3A}, Y^{4A}, and Y^{5A} are hydrogen; four of Y^{6A}, Y^{7A}, Y^{8A}, Y^{9A}, Y^{10A}, Y^{11A}, Y^{12A}, Y^{13A} ,Y^{14A}, and Y^{15A} are deuterium; and the remainder of Y^{6A}, Y^{7A}, Y^{8A}, Y^{9A}, Y^{10A}, Y^{11A}, Y^{12A}, Y^{13A} ,Y^{14A}, and Y^{15A} are hydrogen. In yet other instances, each of Y^{1A}, Y^{2A}, Y^{3A}, Y^{4A}, and Y^{5A} are hydrogen; six of Y^{6A}, Y^{7A}, Y^{8A}, Y^{9A}, Y^{10A}, Y^{11A}, Y^{12A}, Y^{13A} ,Y^{14A}, and Y^{15A} are deuterium; and the remainder of Y^{6A}, Y^{7A}, Y^{8A}, Y^{9A}, Y^{10A}, Y^{11A}, Y^{12A}, Y^{13A} ,Y^{14A}, and Y^{15A} are hydrogen. Additional examples of particular combinations of substitution at Y^{1A}-Y^{13A} are provided in Table 1.

**Table 1**

| **Y^{1A}** | **Y^{2A}** | **Y^{3A}** | **Y^{4A}** | **Y^{5A}** | **Y^{6A}** | **Y^{7A}** | **Y^{8A}** | **Y^{9A}** | **Y^{10A}** | **Y^{11A}** | **Y^{12A}** | **Y^{13A}** |
|---|---|---|---|---|---|---|---|---|---|---|---|---|
| D | D | D | D | D | D | D | D | D | D | D | D | D |
| H | H | H | D | D | D | D | D | D | D | D | D | D |
| H | H | H | H | H | D | D | D | D | D | D | D | D |
| H | H | H | H | H | H | H | D | D | D | D | D | D |
| H | H | H | H | H | H | H | H | H | D | D | D | D |
| D | D | D | D | D | D | D | D | D | D | D | H | H |
| D | D | D | D | D | D | D | D | D | H | H | H | H |
| D | D | D | D | D | D | D | H | H | H | H | H | H |
| H | H | H | H | D | D | D | D | D | D | D | D | D |
| H | H | H | H | H | H | D | D | D | D | D | D | D |
| H | H | H | H | H | H | H | H | D | D | D | D | D |
| H | H | H | H | H | H | H | H | H | H | D | D | D |
| D | D | D | D | D | D | D | D | D | D | H | H | H |
| D | D | D | D | D | D | D | D | H | H | H | H | H |
| D | D | D | D | D | D | H | H | H | H | H | H | H |

In some embodiments of Table 1, Y^{14A} and Y^{15A} are both hydrogen. In other embodiments of Table 1, Y^{14A} and Y^{15A} are both deuterium.

In some embodiments, X^{1A} is -C(Y^{14A}Y^{15A})-CH=CH-, -CH₂-, -CD₂-, or a direct bond. In some embodiments, X^{2A} is -CH₂CH₂-, -CH₂CD₂-,-CD₂CD₂-, or -CH=CH-. A variety of specific combinations of X^{1A} and X^{2A} are contemplated herein. These combinations are shown in Table 2.

**Table 2**

| **X^{1A}** | **X^{2A}** |
|---|---|
| -CD₂-CH=CH- | -CH₂CH₂- |
| -CD₂-CH=CH- | -CH₂CD₂- |
| -CD₂-CH=CH- | -CD₂CH₂- |
| -CD₂-CH=CH- | -CD₂CD₂- |
| -CD₂-CH=CH- | -CH=CH- |
| -CD₂-CH=CH- | -CH₂CH₂- |
| -CD₂-CH=CH- | -CH₂CD₂- |
| -CD₂-CH=CH- | -CD₂CH₂- |
| -CD₂-CH=CH- | -CD₂CD₂- |
| -CD₂-CH=CH- | -CH=CH- |

| **X^{1A}** | **X^{2A}** |
|---|---|
| -CH₂- | -CH₂CH₂- |
| -CH₂- | -CH₂CD₂- |
| -CH₂- | -CD₂CH₂- |
| -CH₂- | -CD₂CD₂- |
| -CH₂- | -CH=CH- |
| -CD₂- | -CH₂CH₂- |
| -CD₂- | -CH₂CD₂- |
| -CD₂- | -CD₂CH₂- |
| -CD₂- | -CD₂CD₂- |
| -CD₂- | -CH=CH- |
| Direct bond | -CH₂CH₂- |
| Direct bond | -CH₂CD₂- |
| Direct bond | -CD₂CH₂- |
| Direct bond | -CD₂CD₂- |
| Direct bond | -CH=CH- |

In some embodiments, R^{1A} is a substituted or unsubstituted -O-C₁-C₆ alkyl, a substituted or unsubstituted -S-C₁-C₆ alkyl, a substituted or unsubstituted -NH-C₁-C₆ alkyl, -NH₂, -OH, an unsubstituted sphingolipid, an unsubstituted glyceryl ester, or 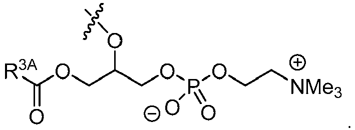 In some embodiments, R^{1A} is

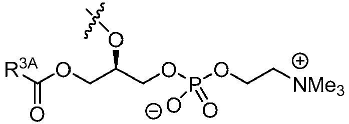

In some embodiments, R^{1A} is an unsubstituted -O-C₁-C₆ alkyl, such as -OCH₃, -OCH₂CH₃, -O-nPr, -O-iPr, -O-nBu, -O-sBu, -O-tBu, -O-pentyl (straight chain or branched), and -O-hexyl (straight chain or branched). In some instances, R^{1A} is -OCH₃ or -OCH₂CH₃. In other instances, R^{1A} is -OCH₂CH₃. In other embodiments, R^{1A} is an unsubstituted -O-C₁-C₆ alkyl, such as those described herein.

In some embodiments, R^{1A} is an unsubstituted -S-C₁-C₆ alkyl, such as -SCH₃,-SCH₂CH₃, -S-nPr, -S-iPr, -S-nBu, -S-sBu, -S-tBu, -S-pentyl (straight chain or branched), and -S-hexyl (straight chain or branched). In other embodiments, R^{1A} is an unsubstituted -S-C₁-C₆ alkyl, such as those described herein.

In some embodiments, R^{1A} is an unsubstituted -NH-C₁-C₆ alkyl, such as -NHCH₃, -NHCH₂CH₃, -NH-nPr, -NH-iPr, -NH-nBu, -NH-sBu, -NH-tBu, -O-pentyl (straight chain or branched), and -NH-hexyl (straight chain or branched). In other embodiments, R^{1A} is an unsubstituted -NH-C₁-C₆ alkyl, such as those described herein.

In some embodiments, R^{1A} is -NH₂. In other embodiments, R^{1A} is -OH.

In some embodiments, R^{1A} is an unsubstituted sphingolipid, such as a ceramide, a dihydroceramide, a sphingomyelin, a cerebroside, a sulfatide, or a ganglioside.

In some embodiments, R^{1A} is an unsubstituted glyceryl ester, such as an unsubstituted mono-glyceryl ester; an unsubstituted di-glyceryl ester; and an unsubstituted triglyceryl ester. In some instances, R^{1A} is an unsubstituted glyceryl ester selected from phosphatidylethanolamines, phosphatidylcholines, phosphatidylserines, and phosphoinositides; for example, acyl 1,3-dihydroxypropan-2-yl.

In some embodiments, R^{1A} is 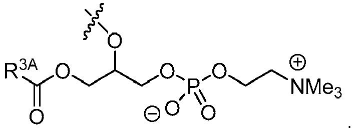 In some further embodiments, R^{1A} is 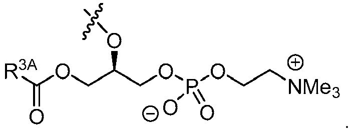 In some such embodiments, R^{3A} is an unsubstituted C₁-C₂₀ alkyl, such as those described herein. In some instances, R^{3A} is an unsubstituted C₇-C₁₉ alkyl, C₉-C₁₇ alkyl, C₁₁-C₁₅ alkyl, or C₁-C₆ alkyl, such as those described herein.

In some embodiments, at least two Y^{1A}-Y^{15A} are not hydrogen.

In some embodiments, Formula (II) is not selected from: 7,7,10,10,13,13,16,16-D₈-eicosapentaenoic acid, or a salt or ester thereof; 13,13,16,16-D₄-eicosapentaenoic acid, or a salt or ester thereof; 19,19,20,20,20-D₅-eicosapentaenoic acid, or a salt or ester thereof; 21,21,22,22,22-D₅-docosahexaenoic acid, or a salt or ester thereof; 6,6,9,9,12,12,15,15,18,18-D₁₀-docosahexaenoic acid, or a salt or ester thereof; or 7,7,10,10,13,13-D₆-arachadonic acid, or a salt or ester thereof. In other words, compounds of Formula (II) do not include the specific compounds, salts, and/or esters disclosed in this paragraph.

In some embodiments, the compound of Formula (II), has the structure of Formula (IIA): 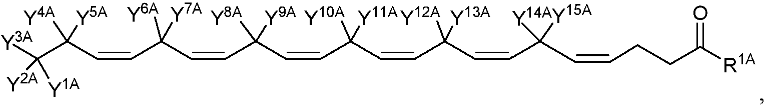 or a pharmaceutically acceptable salt thereof. In some such embodiments, each of Y^{6A}, Y^{7A}, Y^{8A}, Y^{9A}, Y^{10A}, Y^{11A}, Y^{12A}, Y^{13A}, Y^{14A}, and Y^{15A} is D. In some further embodiments, each of Y^{1A}, Y^{2A}, Y^{3A}, Y^{4A}, and Y^{5A} is H. In some other embodiments, at least one of Y^{1A}, Y^{2A}, Y^{3A}, Y^{4A}, and Y^{5A} is D. In some other embodiments, R^{1A} may contain one or more deuterium atoms. In some further embodiments, the compound may further be deuterated at a pro-bis-allylic position.

In other embodiments, the compound of Formula (II), has the structure of Formula (IIB): 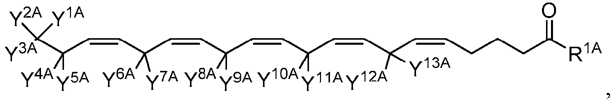 or a pharmaceutically acceptable salt thereof. In some such embodiments, each of each of Y^{6A}, Y^{7A}, Y^{8A}, Y^{9A}, Y^{10A}, Y^{11A}, Y^{12A} and Y^{13A} is D. In some further embodiments, each of Y^{1A}, Y^{2A}, Y^{3A}, Y^{4A}, and Y^{5A} is H. In some other embodiments, at least one of Y^{1A}, Y^{2A}, Y^{3A}, Y^{4A}, and Y^{5A} is D. In some other embodiments, R^{1A} may contain one or more deuterium atoms. In some further embodiments, the compound may further be deuterated at a pro-bis-allylic position.

In still other embodiments, the compound of Formula (II), has the structure of Formula (IIC): 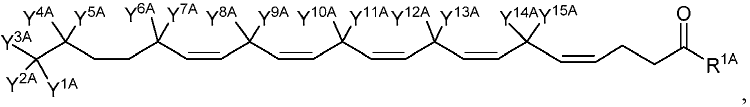 or a pharmaceutically acceptable salt thereof. In some such embodiments, each of Y^{8A}, Y^{9A}, Y^{10A}, Y^{11A}, Y^{12A}, Y^{13A}, Y^{14A}, and Y^{15A} is D. In some further embodiments, each of Y^{1A}, Y^{2A}, Y^{3A}, Y^{4A}, Y^{5A}, Y^{6A} and Y^{7A} is H. In some other embodiments, at least one of Y^{1A}, Y^{2A}, Y^{3A}, Y^{4A}, Y^{5A}, Y^{6A} and Y^{7A} is D. In some embodiments, the ethylene linker between -C(Y^{4A}Y^{5A})- and -C(Y^{6A}Y^{7A})-may be substituted with one or more D. In some other embodiments, R^{1A} may contain one or more D. In some further embodiments, the compound may further be deuterated at a pro-bis-allylic position.

In yet other embodiments, the compound of Formula (II), has the structure of Formula (IID): 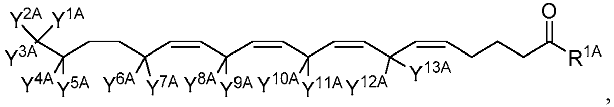 or a pharmaceutically acceptable salt thereof. In some such embodiments, each of Y^{8A}, Y^{9A}, Y^{10A}, Y^{11A}, Y^{12A} and Y^{13A} is D. In some further embodiments, each of Y^{1A}, Y^{2A}, Y^{3A}, Y^{4A}, Y^{5A}, Y^{6A} and Y^{7A} is H. In some other embodiments, at least one of Y^{1A}, Y^{2A}, Y^{3A}, Y^{4A}, Y^{5A}, Y^{6A} and Y^{7A} is D. In some embodiments, the ethylene linker between -C(Y^{4A}Y^{5A})- and -C(Y^{6A}Y^{7A})- may be substituted with one or more D. In some other embodiments, R^{1A} may contain one or more deuterium atoms. In some further embodiments, the compound may further be deuterated at a pro-bis-allylic position.

### Methods

In some embodiments, it is unnecessary to substitute all isotopically unmodified PUFAs (i.e., non-deuterated, endogenous H-PUFAs) with isotopically modified PUFAs (such as compounds of Formula (I), compounds of Formula (II), or pharmaceutically acceptable salts of any of the foregoing). In some embodiments, is preferable to have sufficient levels of compounds of Formula (I), compounds of Formula (II), or pharmaceutically acceptable salts of any of the foregoing, in the membrane to prevent unmodified PUFAs such as H-PUFAs from sustaining a chain reaction of self-oxidation. During self-oxidation, when one PUFA oxidizes, and there is a non-oxidized PUFA in the vicinity, the non-oxidized PUFA can get oxidized by the oxidized PUFA. This may also be referred to as autooxidation. In some instances, if there is a low concentration, for example "dilute" H-PUFAs in the membrane with D-PUFAs, this oxidation cycle may be broken due to the distance separating H-PUFAs. In some embodiments, the concentration of compounds of Formula (I), compounds of Formula (II), or pharmaceutically acceptable salts of any of the foregoing, in the membrane is sufficient amount to break the autooxidation chain reaction. To break the autooxidation chain reaction, for example, 1-60%, 5-50%, or 15-35% of the total molecules of the same type are in the membrane. This may be measured, for example, by IRMS (isotope ratio mass spectrometry).

It has been surprisingly discovered that in some instances, even substituting a very small amount of naturally occurring PUFAs (H-PUFAs) with a deuterated PUFA described herein may be sufficient to break the autooxidation chain reaction. In some instance, the presence of less than about 10%, less than about 5%, less than 2% or even less than 1% of a deuterated PUFA described herein in the membrane can reduce or prevent lipid autooxidation and also may be used to treat of ameliorate various diseases or conditions described herein (such as ferrosis, a retinal condition, as described herein), where the underlying causes is due to lipid autooxidation or peroxidation. In some embodiments, the deuterated compound is a compound of Formula (I) or Formula (II), or a pharamceuticall acceptable salt thereof. In some embodiments, the compound is a deuterated polyunsaturated fatty acid PUFA , such as omega 3 PUFA or omega 6 PUFA. In some further embodiments, the compound is a deuterated arachidonic acid, a deuterated eicosapentaenoic acid, or a deuterated docosahexaenoic acid, or a salt or ester (such as alkyl ester) thereof. In some further embodiments, the deterated PUFA described herein is deuterated at all bis-allylic positions (such as 7,7,10,10,13,13-D6-arachidonic acid; 7,7,10,10,13,13,16,16-D8-eicosapentaenoic acid, or 6,6,9,9,12,12,15,15,18,18-D10-docosahexanoic acid or a salt or ester thereof). In some further embodiment, the deuterated PUFA described herein may further contain deuterium at one or more pro-bis-allylic position.

Some embodiments provide a method of reducing lipid autooxidation in a subject in need thereof, comprising administering an effective amount of a compound of Formula (I), or a pharmaceutically acceptable salt thereof, to the subject: 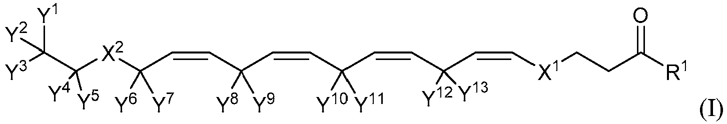 or a pharmaceutically acceptable salt thereof, wherein: each of Y¹, Y², Y³, Y⁴, Y⁵, Y⁶, Y⁷, Y⁸, Y⁹, Y¹⁰, Y¹¹, Y¹², Y¹³ ,Y¹⁴, and Y¹⁵ are independently hydrogen and deuterium; X¹ is -C(Y¹⁴Y¹⁵)-CH=CH-, -CH₂-, -CD₂-, or a direct bond; X² is -CH₂CH₂-, -CH₂CD₂-, -CD₂CH₂-, -CD₂CD₂-, or -CH=CH-; R¹ is a substituted or unsubstituted -O-C₁-C₆ alkyl, a substituted or unsubstituted -S-C₁-C₆ alkyl, a substituted or unsubstituted -NH-C₁-C₆ alkyl, -NH₂, -OH, an unsubstituted sphingolipid, or an unsubstituted glyceryl ester; wherein at least two of Y¹, Y², Y³, Y⁴, Y⁵, Y⁶, Y⁷, Y⁸, Y⁹, Y¹⁰, Y¹¹, Y¹², Y¹³ ,Y¹⁴, and Y¹⁵ are independently deuterium; wherein, when each of Y¹-Y⁵ are deuterium, then X¹ is -C(Y¹⁴Y¹⁵)-CH=CH- and at least one of Y⁶-Y¹⁵ is deuterium; or X¹ is -CD₂-; or X² is -CH₂CD₂-, -CD₂CH₂-, or -CD₂CD₂-; and wherein the compound of Formula (I), or a pharmaceutically acceptable salt thereof, comprises from about 1% to about 99% of the total amount of fats, fatty acids, and fatty acid esters administered to, or ingested by, the subject. Other embodiments provide a method of reducing lipid autooxidation in a subject in need thereof, comprising administering an effective amount of a compound of Formula (II), or a pharmaceutically acceptable salt thereof, as described herein, to the subject; wherein the compound of Formula (I), or a pharmaceutically acceptable salt thereof, comprises from about 1% to about 99% of the total amount of fats, fatty acids, and fatty acid esters administered to, or ingested by, the subject. In some embodiments, the lipid autooxidation is reduced by about 10%, 20%, 30%, 40%, 50%, 60%, 70%, 80%, 90%, 95%, 99%, or any value in between these numbers.

Some embodiments provide a method of reducing ferroptosis in a subject in need thereof, comprising administering an effective amount of a compound of Formula (I), or a pharmaceutically acceptable salt thereof, as described herein, to the subject wherein the compound of Formula (I), or a pharmaceutically acceptable salt thereof, comprises from about 1% to about 99% of the total amount of fats, fatty acids, and fatty acid esters administered to, or ingested by, the subject. Other embodiments provide a method of reducing ferroptosis in a subject in need thereof, comprising administering an effective amount of a compound of Formula (II), or a pharmaceutically acceptable salt thereof, as described herein, to the subject wherein the compound of Formula (II), or a pharmaceutically acceptable salt thereof, comprises from about 1% to about 99% of the total amount of fats, fatty acids, and fatty acid esters administered to, or ingested by, the subject.

The present invention provides a method of treating or ameliorating a retinal condition in a subject in need thereof, comprising administering an effective amount of a compound of Formula (I), or a pharmaceutically acceptable salt thereof, to the subject wherein the compound of Formula (I), or a pharmaceutically acceptable salt thereof, comprises from about 1% to about 99% of the total amount of fats, fatty acids, and fatty acid esters administered to, or ingested by, the subject; and wherein the retinal condition is selected from Leber's hereditary optic neuropathy (LHON), macular telangiectasia, and glaucoma. Other embodiments provide a method of treating or ameliorating a retinal condition in a subject in need thereof, comprising administering an effective amount of a compound of Formula (II), or a pharmaceutically acceptable salt thereof, to the subject wherein the compound of Formula (II), or a pharmaceutically acceptable salt thereof, comprises from about 1% to about 99% of the total amount of fats, fatty acids, and fatty acid esters administered to, or ingested by, the subject; and wherein the retinal condition is glaucoma.

In some embodiments, the retinal condition is glaucoma. In some embodiments, the retinal condition is Leber's hereditary optic neuropathy (LHON). In some embodiments, the retinal condition is macular telangiectasia.

In any embodiments of the methods described herein, the compound of Formula (I), or a pharmaceutically acceptable salt thereof, comprises less than about 5% of the total amount of fats, fatty acids, and fatty acid esters administered to, or ingested by, the subject. In some such embodiments, the compound of Formula (I), or a pharmaceutically acceptable salt thereof, comprises from about 0.5%, 1%, 1.5%, 2%, 2.5%, 3%, 3.5%, 4%, or 4.5% (or a range defined by any two of the preceding values, such as 0.5% to 4.5%, 1% to 4%, 1.5% to 3.5%, or 2% to 3%) of the total amount of fats, fatty acids, and fatty acid esters administered to, or ingested by, the subject. In some further embobdiments, the compound of Formula (I), or a pharmaceutically acceptable salt thereof, comprises less than about 2% or less than about 1% of the total amount of fats, fatty acids, and fatty acid esters administered to, or ingested by, the subject.

In some embodiments, the compound of Formula (I), has the structure of Formula (IA): 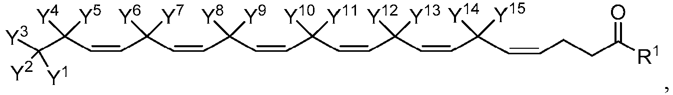 or a pharmaceutically acceptable salt thereof. In some such embodiments, each of Y⁶, Y⁷, Y⁸, Y⁹, Y¹⁰, Y¹¹, Y¹², Y¹³, Y¹⁴, and Y¹⁵ is D. In some further embodiments, each of Y¹, Y², Y³, Y⁴, and Y⁵ is H. In some other embodiments, at least one of Y¹, Y², Y³, Y⁴, and Y⁵ is D. In some other embodiments, R¹ may contain one or more deuterium atoms. In some further embodiments, the compound may further be deuterated at a pro-bis-allylic position.

In other embodiments, the compound of Formula (I), has the structure of Formula (IB): 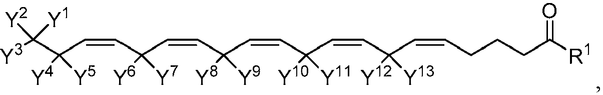 or a pharmaceutically acceptable salt thereof. In some such embodiments, each of Y⁶, Y⁷, Y⁸, Y⁹, Y¹⁰, Y¹¹, Y¹², and Y¹³ is D. In some further embodiments, each of Y¹, Y², Y³, Y⁴, and Y⁵ is H. In some other embodiments, at least one of Y¹, Y², Y³, Y⁴, and Y⁵ is D. In some other embodiments, R¹ may contain one or more deuterium atoms. In some further embodiments, the compound may further be deuterated at a pro-bis-allylic position.

In still other embodiments, the compound of Formula (I), has the structure of Formula (IC): 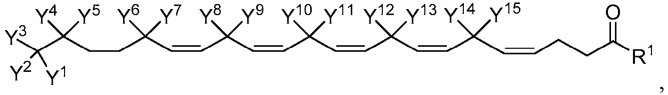 or a pharmaceutically acceptable salt thereof. In some such embodiments, each of Y⁸, Y⁹, Y¹⁰, Y¹¹, Y¹², Y¹³, Y¹⁴, and Y¹⁵ is D. In some further embodiments, each of Y¹, Y², Y³, Y⁴, Y⁵, Y⁶ and Y⁷ is H. In some other embodiments, at least one of Y¹, Y², Y³, Y⁴, Y⁵, Y⁶ and Y⁷ is D. In some embodiments, the ethylene linker between -C(Y⁴Y⁵)- and -C(Y⁶Y⁷)- may be substituted with one or more D. In some other embodiments, R¹ may contain one or more D. In some further embodiments, the compound may further be deuterated at a pro-bis-allylic position.

In yet other embodiments, the compound of Formula (I), has the structure of Formula (ID): 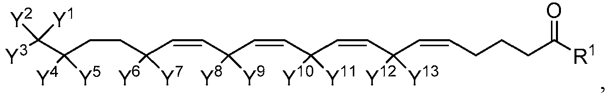 or a pharmaceutically acceptable salt thereof. In some such embodiments, each of Y⁸, Y⁹, Y¹⁰, Y¹¹, Y¹² and Y¹³ is D. In some further embodiments, each of Y¹, Y², Y³, Y⁴, Y⁵, Y⁶ and Y⁷ is H. In some other embodiments, at least one of Y¹, Y², Y³, Y⁴, Y⁵, Y⁶ and Y⁷ is D. In some embodiments, the ethylene linker between -C(Y⁴Y⁵)- and -C(Y⁶Y⁷)- may be substituted with one or more D. In some other embodiments, R¹ may contain one or more deuterium atoms. In some further embodiments, the compound may further be deuterated at a pro-bis-allylic position.

In some embodiments of the compounds of Formula (I), (IA), (IB), (IC) or (ID), R¹ is an unsubstituted -O-C₁-C₆ alkyl, such as those described herein. In some embodiments, R¹ is -OCH₃ or -OCH₂CH₃. In some instances, R¹ is -OCH₂CH₃.

In some embodiments of the compounds of Formula (I), (IA), (IB), (IC) or (ID), R¹ is a substituted -O-C₁-C₆ alkyl, such as those described herein. In other embodiments, R¹ is a substituted or unsubstituted -S-C₁-C₆ alkyl, such as those described herein. In still other embodiments, R¹ is a substituted or unsubstituted -NH-C₁-C₆ alkyl, such as those described herein. In some embodiments, R¹ is -NH₂. In other embodiments, R¹ is -OH.

In some embodiments of the compounds of Formula (I), (IA), (IB), (IC) or (ID), R¹ is an unsubstituted glyceryl ester, for example, an unsubstituted monoglycerylyl ester; an unsubstituted diglyceryl ester; or an unsubstituted triglyceryl ester. In some embodiments, R¹ is an unsubstituted glyceryl ester selected from phosphatidylethanolamines, phosphatidylcholines, phosphatidylserines, phosphoinositides, ceramides, and sphingomyelins.

In some embodiments of the compounds of Formula (I), (IA), (IB), (IC) or (ID), each of Y¹, Y², Y³, Y⁴, Y⁵, Y⁶, Y⁷, Y⁸, Y⁹, Y¹⁰, Y¹¹, Y¹², Y¹³ ,Y¹⁴, and Y¹⁵ are deuterium.

In other aspects, various combinations of Y¹, Y², Y³, Y⁴, and Y⁵ are hydrogen; and each of Y⁶, Y⁷, Y⁸, Y⁹, Y¹⁰, Y¹¹, Y¹², and Y¹³ being independently hydrogen or deuterium, are contemplated herein. For example, in some embodiments, each of Y¹, Y², Y³, Y⁴, and Y⁵ are hydrogen; and each of Y⁶, Y⁷, Y⁸, Y⁹, Y¹⁰, Y¹¹, Y¹², Y¹³ ,Y¹⁴, and Y¹⁵ are deuterium. In other embodiments, each of Y¹, Y², Y³, Y⁴, Y⁵, Y⁶, and Y⁷ are hydrogen; and Y⁸, Y⁹, Y¹⁰, Y¹¹, Y¹², Y¹³, Y¹⁴, and Y¹⁵ are deuterium. In still other embodiments, each of Y¹, Y², Y³, Y⁴, Y⁵, Y⁶, Y⁷, Y⁸ and Y⁹ are hydrogen; and Y¹⁰, Y¹¹, Y¹², Y¹³ ,Y¹⁴, and Y¹⁵ are deuterium. In some instances, each of Y¹, Y², Y³, Y⁴, Y⁵ Y⁶, Y⁷, Y⁸, Y⁹, Y¹⁰ and Y¹¹ are hydrogen; and Y¹², Y¹³ ,Y¹⁴, and Y¹⁵ are deuterium. In other instances, each of Y¹, Y², Y³, Y⁴, and Y⁵ are hydrogen; two of Y⁶, Y⁷, Y⁸, Y⁹, Y¹⁰, Y¹¹, Y¹², Y¹³ ,Y¹⁴, and Y¹⁵ are deuterium; and the remainder of Y⁶, Y⁷, Y⁸, Y⁹, Y¹⁰, Y¹¹, Y¹², Y¹³ ,Y¹⁴, and Y¹⁵ are hydrogen. In some embodiments, each of Y¹, Y², Y³, Y⁴, and Y⁵ are hydrogen; four of Y⁶, Y⁷, Y⁸, Y⁹, Y¹⁰, Y¹¹, Y¹², Y¹³ ,Y¹⁴, and Y¹⁵ are deuterium; and the remainder of Y⁶, Y⁷, Y⁸, Y⁹, Y¹⁰, Y¹¹, Y¹², Y¹³ ,Y¹⁴, and Y¹⁵ are hydrogen. In other embodiments, each of Y¹, Y², Y³, Y⁴, and Y⁵ are hydrogen; six of Y⁶, Y⁷, Y⁸, Y⁹, Y¹⁰, Y¹¹, Y¹², Y¹³ ,Y¹⁴, and Y¹⁵ are deuterium; and the remainder of Y⁶, Y⁷, Y⁸, Y⁹, Y¹⁰, Y¹¹, Y¹², Y¹³ Y¹⁴, and Y¹⁵ are hydrogen. Additional examples of particular combinations of substitution at Y¹-Y¹³ are provided in Table 3.

**Table 3**

| **Y¹** | **Y²** | **Y³** | **Y⁴** | **Y⁵** | **Y⁶** | **Y⁷** | **Y⁸** | **Y⁹** | **Y¹⁰** | **Y¹¹** | **Y¹²** | **Y¹³** |
|---|---|---|---|---|---|---|---|---|---|---|---|---|
| D | D | D | D | D | D | D | D | D | D | D | D | D |
| H | H | H | D | D | D | D | D | D | D | D | D | D |
| H | H | H | H | H | D | D | D | D | D | D | D | D |
| H | H | H | H | H | H | H | D | D | D | D | D | D |
| H | H | H | H | H | H | H | H | H | D | D | D | D |
| D | D | D | D | D | D | D | D | D | D | D | H | H |

| **Y¹** | **Y²** | **Y³** | **Y⁴** | **Y⁵** | **Y⁶** | **Y⁷** | **Y⁸** | **Y⁹** | **Y¹⁰** | **Y¹¹** | **Y¹²** | **Y¹³** |
|---|---|---|---|---|---|---|---|---|---|---|---|---|
| D | D | D | D | D | D | D | D | D | H | H | H | H |
| D | D | D | D | D | D | D | H | H | H | H | H | H |
| H | H | H | H | D | D | D | D | D | D | D | D | D |
| H | H | H | H | H | H | D | D | D | D | D | D | D |
| H | H | H | H | H | H | H | H | D | D | D | D | D |
| H | H | H | H | H | H | H | H | H | H | D | D | D |
| D | D | D | D | D | D | D | D | D | D | H | H | H |
| D | D | D | D | D | D | D | D | H | H | H | H | H |
| D | D | D | D | D | D | H | H | H | H | H | H | H |

In some embodiments of Table 3, Y¹⁴ and Y¹⁵ are both hydrogen. In other embodiments of Table 3, Y¹⁴ and Y¹⁵ are both hydrogen.

In some embodiments, X¹ is -C(Y¹⁴Y¹⁵)-CH=CH-. In some embodiments, X¹ is -CH₂-. In still other embodiments, X¹ is -CD₂-. In yet other embodiments, X¹ is a direct bond. When X¹ is a direct bond, the methylene group beta to the carbonyl group (attached to R¹) in Formula (I) is at the allylic position relative to the right-most double bond in Formula (I) (as shown herein).

In some embodiments, X² is -CH₂CH₂-. In other embodiments, X² is -CH₂CD₂-. In still other embodiments, X² is -CD₂CH₂-. In some embodiments, X² is -CD₂CD₂-. In other embodiments, X² is or -CH=CH-. Selected specific combinations of X¹ and X² are shown in Table 4.

**Table 4.**

| **X¹** | **X²** |
|---|---|
| -CD₂-CH=CH- | -CH₂CH₂- |
| -CD₂-CH=CH- | -CH₂CD₂- |
| -CD₂-CH=CH- | -CD₂CH₂- |
| -CD₂-CH=CH- | -CD₂CD₂- |
| -CD₂-CH=CH- | -CH=CH- |

| **X¹** | **X²** |
|---|---|
| -CD₂-CH=CH- | -CH₂CH₂- |
| -CD₂-CH=CH- | -CH₂CD₂- |
| -CD₂-CH=CH- | -CD₂CH₂- |
| -CD₂-CH=CH- | -CD₂CD₂- |
| -CD₂-CH=CH- | -CH=CH- |

| **X**¹ | **X²** |
|---|---|
| -CH₂- | -CH₂CH₂- |
| -CH₂- | -CH₂CD₂- |
| -CH₂- | -CD₂CH₂- |
| -CH₂- | -CD₂CD₂- |
| -CH₂- | -CH=CH- |

| **X**¹ | **X²** |
|---|---|
| -CD₂- | -CH₂CH₂- |
| -CD₂- | -CH₂CD₂- |
| -CD₂- | -CD₂CH₂- |
| -CD₂- | -CD₂CD₂- |
| -CD₂- | -CH=CH- |
| Direct bond | -CH₂CH₂- |
| Direct bond | -CH₂CD₂- |
| Direct bond | -CD₂CH₂- |
| Direct bond | -CD₂CD₂- |
| Direct bond | -CH=CH- |

In some embodiments of the compound of Formula (I) described herein, the compound is not a deuterated arachidonic acid, or a salt or alkyl ester thereof, such as D₃, D₄, D₅, or D₆-Ara (i.e., Ara having 3, 4, 5 or 6 deterium at bis-allylic positions).

Varying amounts of a compounds of Formula (I), compounds of Formula (II), or pharmaceutically acceptable salts of any of the foregoing, can be administered to, or ingested by, subjects in need thereof. In addition to measuring the total amount administered or ingested, the amount of compounds of Formula (I), compounds of Formula (II), or pharmaceutically acceptable salts of any of the foregoing, administered to, or ingested by, a subject in need thereof, is by measuring the percent of the total amount of fats, fatty acids, and fatty acid esters administered to, or ingested by, the subject made up by a compounds of Formula (I), compounds of Formula (II), or pharmaceutically acceptable salts of any of the foregoing. For example, in some embodiments, the compounds of Formula (I), compounds of Formula (II), or pharmaceutically acceptable salts of any of the foregoing, comprises from about 0.5% to about 1.5% of the total amount of fats, fatty acids, and fatty acid esters administered to, or ingested by, the subject, such as about 1%. In other embodiments, the compounds of Formula (I), compounds of Formula (II), or pharmaceutically acceptable salts of any of the foregoing, comprises from about 1% to about 95%; about 1% to about 90%; about 1% to about 75%; about 1% to about 50%; about 1% to about 25%; about 1% to about 10%; about 1% to about 5%; about 1% to about 2.5%; or about 1% to about 2%, of the total amount of fats, fatty acids, and fatty acid esters administered to, or ingested by, the subject. In some other embodiments, the compounds of Formula (I), compounds of Formula (II), or pharmaceutically acceptable salts thereof may also comprises less than about 1%, 0.9%, 0,8%, 0.7%, 0.6%, or 0.5% (for example, about 0.5% to about 1%, about 0.6% to about 0.9%, or about 0.7% to about 0.8%) of the total amount of fats, fatty acids, and fatty acid esters administered to, or ingested by, the subject.

In some embodiments, the amount of the compounds of Formula (I), compounds of Formula (II), or pharmaceutically acceptable salts of any of the foregoing, administered to, or ingested by, the subject provides a total isotopic load administered to, or ingested by, the subject that is in the range of from about 1% to about 99%. For example, in various embodiments the amount of the compounds of Formula (I), compounds of Formula (II), or pharmaceutically acceptable salts of any of the foregoing, administered to, or ingested by, the subject provides a total isotopic load administered to, or ingested by, the subject that is in the range of from about 1% to about 50%; about 1% to about 20%; about 1% to about 10%; or about 1% to about 5%.

In some embodiments, the compound of Formula (I), or a pharmaceutically acceptable salt thereof, is selected from: 7,7,10,10,13,13,16,16-D₈-eicosapentaenoic acid, or a salt or thereof: 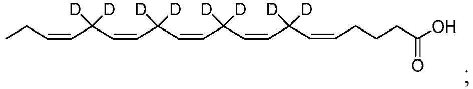 6,6,9,9,12,12,15,15,18,18-D₁₀-docosahexaenoic acid, or a salt thereof: 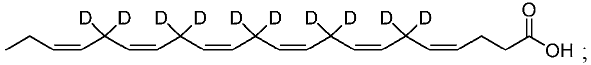 7,7,10,10,13,13,16,16-D₈-eicosapentaenoic acid ethyl ester: 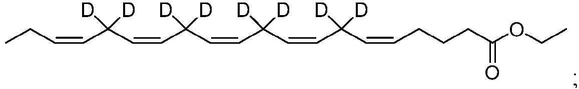 or 6,6,9,9,12,12,15,15,18,18-D₁₀-docosahexaenoic acid ethyl ester: 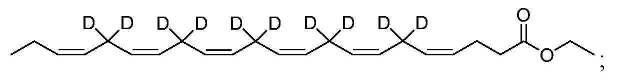 ; or a combination of any of the foregoing. In some instances, the compound of Formula (I), or a pharmaceutically acceptable salt thereof, is selected from one of the compounds described in this paragraph, or a pharmaceutically acceptable salt thereof. In other instances, the compound of Formula (I), or a pharmaceutically acceptable salt thereof, is selected from two of the compounds described in this paragraph, or a pharmaceutically acceptable salt thereof; or from three of the compounds described in this paragraph, or a pharmaceutically acceptable salt thereof. In still other instances, the compound of Formula (I), or a pharmaceutically acceptable salt thereof, is a combination of all the compounds described in this paragraph.

Various indicators for determining the effectiveness of a method for treating retinal conditions are known to those skilled in the art. Example of suitable indicators include, but are not limited to, an increase in visual acuity, a reduction in eye pain, an increase in color perception,
and/or other indicator of disease response.

In some embodiments, a compounds of Formula (I), compounds of Formula (II), or pharmaceutically acceptable salts of any of the foregoing, can result in at least a 1, 2, 3, 4, 5, 10, 15, 20, 25, 50, 75, 100-fold or more improvement in the aforementioned clinical outcomes relative to pre-treatment levels in a subject, as determined several hours after receiving the initial dosage of the compound (for example, 60 hours after receiving the initial dosage of the compound). In some embodiments, a compounds of Formula (I), compounds of Formula (II), or pharmaceutically acceptable salts of any of the foregoing, can result in at least a 1, 2, 3, 4, 5, 10, 15, 20, 25, 50, 75, 100-fold or more improvement in vision as determined several weeks after receiving the initial dosage of the compound (for example, 10 weeks after receiving the initial dosage of the compound) compared to the improvement in vision achieved by the standard of care.

In some embodiments, a compounds of Formula (I), compounds of Formula (II), or pharmaceutically acceptable salts of any of the foregoing, can decrease the percentage of subjects that experience complications from treatment for one or more of the retinal conditions described herein compared to the percentage of subjects that experience complication being treated with the standard of care for the retinal condition. For example, the percentage of subjects being treated with a compounds of Formula (I), compounds of Formula (II), or pharmaceutically acceptable salts of any of the foregoing, that experience complications can be 10%, 25%, 40%, 50%, 60%, 70%, 80% and 90% less compared to subjects being treated with the standard of care.

In some embodiments, the methods described herein impart an amount of heavy atoms in a particular tissue. Thus, in some aspects, the amount of heavy atoms in the tissue will be a particular percentage of the same type of atoms in the tissue. For example, the number of heavy atoms may be about 1%-100% of the total amount of the same type of atoms. In some aspects, 10-50% the atoms are substituted with the same type of heavy atoms. In some embodiments, the tissue is the eye, including, but not limited to the retina, for example, photoreceptors, the retinal pigment epithelium (RPE), choroid, Bruch's membrane, and retinal ganglion cells.

As will be readily apparent to one skilled in the art, the useful *in vivo* dosage to be administered and the particular mode of administration will vary depending upon the age, weight, the severity of the affliction, and mammalian species treated, the particular compounds employed, and the specific use for which these compounds are employed. The determination of effective dosage levels, that is the dosage levels necessary to achieve the desired result, can be accomplished by one skilled in the art using routine methods, for example, human clinical trials and *in vitro* studies.

The dosage may range broadly, depending upon the desired effects and the therapeutic indication. Alternatively dosages may be based and calculated upon the surface area of the patient, as understood by those of skill in the art. Although the exact dosage will be determined on a drug-by-drug basis, in most cases, some generalizations regarding the dosage can be made. The daily dosage regimen for an adult human patient may be, for example, an oral dose of between 100 mg and 8000 mg of each active ingredient, preferably between 800 mg and 2000 mg, e.g., 1000 mg.

In some embodiments, compounds are dosed at about 0.01 mg/kg to about 1000 mg/kg, about 0.1 mg/kg to about 100 mg/kg, and/or about 1 mg/kg to about 10 mg/kg. In other embodiments, compounds are dosed at about: 0.01, 0.1, 1.0, 5.0, 10, 25, 50, 75, 100, 150, 200, 300, 400, 500, and/or 1000 mg/kg. In some embodiments, compounds are dosed by mouth with a morning, afternoon, and/or evening meal. In some embodiments, 0.25 g, 0.5 g, 0.75 g, 1 g, 1.25 g, 1.5 g, 1.75 g, 2 g, 2.5 g, 3 g, 3.5 g, 4 g, 4.5 g, 5 g, 5.5 g, 6 g, 6.5 g, 7 g, 7.5 g, 8 g, 9 g, 10, or 15 g, or a range bounded by any two of the aforementioned numbers are administered daily. In other embodiments, the aforementioned amounts are dosed at a single meal.

In some embodiments, the desired daily dose is administered over two or more meals. In some embodiments, 1 g, 2 g, 4 g, or 8 g are administered per day. The dosage may be a single one or a series of two or more given in the course of one or more days, as is needed by the subject. In some embodiments, the compounds will be administered for a period of continuous therapy, for example for a week or more, or for months or years.

In instances where human dosages for compounds have been established for at least some condition, those same dosages may be used, or dosages that are between about 0.1% and 500%, more preferably between about 25% and 250% of the established human dosage. Where no human dosage is established, as will be the case for newly-discovered pharmaceutical compositions, a suitable human dosage can be inferred from ED₅₀ or ID₅₀ values, or other appropriate values derived from *in vitro* or *in vivo* studies, as qualified by toxicity studies and efficacy studies in animals.

In cases of administration of a pharmaceutically acceptable salt, dosages may be calculated as the free base. As will be understood by those of skill in the art, in certain situations it may be necessary to administer the compounds disclosed herein in amounts that exceed, or even far exceed, the above-stated, preferred dosage range in order to effectively and aggressively treat particularly aggressive diseases.

Dosage amount and interval may be adjusted individually to provide plasma levels of the active moiety, which are sufficient to maintain the modulating effects, or minimal effective concentration (MEC). The MEC will vary for each compound but can be estimated from *in vitro* data. Dosages necessary to achieve the MEC will depend on individual characteristics and route of administration. However, HPLC assays or bioassays can be used to determine plasma concentrations. Dosage intervals can also be determined using MEC value. Compositions should be administered using a regimen, which maintains plasma levels above the MEC for 10-90% of the time, preferably between 30-90% and most preferably between 50-90%. In cases of local administration or selective uptake, the effective local concentration of the drug may not be related to plasma concentration.

It should be noted that the attending physician would know how to and when to terminate, interrupt, or adjust administration due to toxicity or organ dysfunctions. Conversely, the attending physician would also know to adjust treatment to higher levels if the clinical response were not adequate (precluding toxicity). The magnitude of an administrated dose in the management of the disorder of interest will vary with the severity of the condition to be treated and to the route of administration. The severity of the condition may, for example, be evaluated, in part, by standard prognostic evaluation methods. Further, the dose and perhaps dose frequency, will also vary according to the age, body weight, and response of the individual patient. A program comparable to that discussed above may be used in veterinary medicine.

Compounds disclosed herein can be evaluated for efficacy and toxicity using known methods. For example, the toxicology of a particular compound, or of a subset of the compounds, sharing certain chemical moieties, may be established by determining *in vitro* toxicity towards a cell line, such as a mammalian, and preferably human, cell line. The results of such studies are often predictive of toxicity in animals, such as mammals, or more specifically, humans. Alternatively, the toxicity of particular compounds in an animal model, such as mice, rats, rabbits, or monkeys, may be determined using known methods. The efficacy of a particular compound may be established using several recognized methods, such as *in vitro* methods, animal models, or human clinical trials. When selecting a model to determine efficacy, the skilled artisan can be guided by the state of the art to choose an appropriate model, dose, route of administration and/or regime.

### Compositions

Some embodiments provide a pharmaceutical composition comprising a compound of Formula (I), or a pharmaceutically acceptable salt thereof, and at least one pharmaceutically acceptable excipient. Other embodiments provide a pharmaceutical composition comprising a compound of Formula (II), or a pharmaceutically acceptable salt thereof, and at least one pharmaceutically acceptable excipient.

The term "pharmaceutical composition" refers to a mixture of one or more compounds and/or salts disclosed herein with other chemical components, such as one or more excipients. The pharmaceutical composition facilitates administration of the compound to an organism. Pharmaceutical compositions can also be obtained by reacting compounds with inorganic or organic acids such as hydrochloric acid, hydrobromic acid, sulfuric acid, nitric acid, phosphoric acid, methanesulfonic acid, ethanesulfonic acid, p-toluenesulfonic acid, and salicylic acid. Pharmaceutical compositions will generally be tailored to the specific intended route of administration.

As used herein, an "excipient" refers to essentially inert substances that are added to a pharmaceutical composition to provide, without limitation, bulk, consistency, stability, binding ability, lubrication, disintegrating ability etc., to the composition. For example, stabilizers such as anti-oxidants and metal-chelating agents are excipients. Excipients also include ingredients in a pharmaceutical composition that lack appreciable pharmacological activity but may be pharmaceutically necessary or desirable. For example, to increase the bulk of a potent drug whose mass is too small for manufacture and/or administration. It may also be a liquid for the dissolution of a drug to be administered by injection, ingestion or inhalation. For example, a buffered aqueous solution such as, without limitation, phosphate buffered saline that mimics the pH and isotonicity of human blood.

The pharmaceutical compositions described herein can be administered to a human patient *per se,* or in pharmaceutical compositions where they are mixed with other active ingredients, as in combination therapy, or excipients, or combinations thereof. Proper formulation is dependent upon the route of administration chosen. Techniques for formulation and administration of the compounds described herein are known to those skilled in the art.

The pharmaceutical compositions disclosed herein may be manufactured in a manner that is itself known, e.g., by means of conventional mixing, dissolving, granulating, dragee-making, levigating, emulsifying, suspending, encapsulating, entrapping or tableting processes. Additionally, the active ingredients are contained in an amount effective to achieve its intended purpose. Many of the compounds used in the pharmaceutical combinations disclosed herein may be provided as salts with pharmaceutically compatible counterions.

Multiple techniques of administering a compound, salt and/or composition exist in the art including, but not limited to, oral, rectal, pulmonary, topical (including to both the skin, the eye, and hair, and the finger/toe nails), aerosol, injection, infusion and parenteral delivery, including intramuscular, subcutaneous, intravenous, intramedullary injections, intrathecal, direct intraventricular, intraperitoneal, intranasal and intraocular injections. In some embodiments, a compound of Formula (I), or a pharmaceutically acceptable salt thereof, can be administered orally. Likewise, a compound of Formula (II), or a pharmaceutically acceptable salt thereof, can also be administered orally.

One may also administer the compound, salt and/or composition in a local rather than systemic manner, for example, via injection or implantation of the compound directly into the affected area, often in a depot or sustained release formulation. Furthermore, one may administer the compound in a targeted drug delivery system, for example, in a liposome coated with a tissue-specific antibody. The liposomes will be targeted to and taken up selectively by the organ. For example, intranasal or pulmonary delivery to target a respiratory disease or condition may be desirable.

The compositions may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration. The pack or dispenser may also be accompanied with a notice associated with the container in form prescribed by a governmental agency regulating the manufacture, use, or sale of pharmaceuticals, which notice is reflective of approval by the agency of the form of the drug for human or veterinary administration. Such notice, for example, may be the labeling approved by the U.S. Food and Drug Administration for prescription drugs, or the approved product insert. Compositions that can include a compound and/or salt described herein formulated in a compatible pharmaceutical excipient may also be prepared, placed in an appropriate container, and labeled for treatment of an indicated condition.

In some aspects, a composition comprising a compound of Formula (I), or a pharmaceutically acceptable salt thereof, or a compound of Formula (II), or a pharmaceutically acceptable salt thereof, also contains undeuterated PUFAs. As used herein "isotopic purity" refers to a comparison between a) the relative number of molecules of Formula (I) or Formula (II) (or a pharmaceutically acceptable salt of any of the foregoing), and b) the total molecules of both compounds of Formula (I) or Formula (II) (or a pharmaceutically acceptable salt of any of the foregoing) and undeuterated PUFAs. For example, two molecules of Formula (I), or a pharmaceutically acceptable salt thereof, in a composition containing ninety-eight molecules of undeuterated PUFAs will have an isotopic purity of 2%. The isotopic purity of a composition comprising a compound of Formula (I) or Formula (II) (or a pharmaceutically acceptable salt of any of the foregoing), may vary widely. Indeed, the isotopic purity of the compositions described herein may be from about 10% to about 100%; from about 20% to about 75%; or from about 33% to about 66%; or a value in between any of the aforementioned numbers. In some embodiments, the isotopic purity of the compositions described herein may be about 10%; about 20%; about 30%; about 40%; about 50%; about 60%; about 70%; about 80%; about 90%; about 95%; about 99%; or about 100%.

As used herein, "isotopic load" refers to the number of heavy atoms (e.g., ²H, ³H, and ¹³C) in a molecule out of the total number of atoms of that element in the molecule. For example, the isotopic load of CH₃CH₂CD₂CH₃ is 2/14, or ~14.3%. Likewise, the isotopic load of (¹³CH₃)CH₂CD₂CH₃ is 3/14, or about ~21.4%. The isotopic load of compounds of Formula (I) and Formula (II), and pharmaceutically acceptable salts of any of the foregoing, may vary widely. For example, in some embodiments, the isotopic load can be about 5%, 10%, 15%, 20%, 25%, 30%, 35%, 40%, 45%, 50%, 65%, 60%, 65%, 70%, 75%, 80%, 85%, 90%, 95%, or about 100%, or any value in between the aforementioned numbers. In some embodiments, the compositions comprising a compound of Formula (I) or Formula (II) (or a pharmaceutically acceptable salts of any of the foregoing) have high isotopic purity and low isotopic load. For example, in some embodiments, linoleic acid ethyl ester deuterated at the bis-allylic 11,11 position could have a high (>99% D at pos. 11,11) isotopic purity, but low (2D/36H) isotopic load.

### Co-administration

In some embodiments, one or more compounds of compounds of Formula (I), compounds of Formula (II), or pharmaceutically acceptable salts of any of the foregoing, or composition comprising one or more compounds of compounds of Formula (I), compounds of Formula (II), or pharmaceutically acceptable salts of any of the foregoing, are administered in combination. In some embodiments, the compounds of Formula (I), compounds of Formula (II), or pharmaceutically acceptable salts of any of the foregoing, are administered in approximately similar amounts. In other embodiments, compounds of Formula (I), compounds of Formula (II), or pharmaceutically acceptable salts of any of the foregoing, are administered in differing amounts. For example, any one of two or more compounds of Formula (I), compounds of Formula (II), or pharmaceutically acceptable salts of any of the foregoing, in a mixture may represent about 1% to about 99% of a mixture, about 5% to about 95% of a mixture, about 10% to about 90% of a mixture, about 15% to about 85% of a mixture, about 20% to about 80% of a mixture, about 25% to about 75% of a mixture, about 30% to about 70% of a mixture, about 35% to about 65% of a mixture, about 40% to about 60% of a mixture, about 40% to about 60% of a mixture, about 45% to about 55% of a mixture, and/or about 50% of a mixture. In other embodiments, any one of two or more compounds of Formula (I), compounds of Formula (II), or pharmaceutically acceptable salts of any of the foregoing, in a mixture may represent about: 5%, 10%, 15%, 20%, 25%, 30%, 35%, 40%, 45%, 50%, 65%, 60%, 65%, 70%, 75%, 80%, 85%, 90%, 95%, or 100% of a mixture, or a range bounded by any two of the aforementioned numbers.

In some embodiments, a compound of Formula (I), or a pharmaceutically acceptable salt thereof, or composition comprising a compound of Formula (I), or a pharmaceutically acceptable salt thereof, is administered before, concurrently, or after administration of one or more additional therapeutic agents. In some embodiments, the one or more additional therapeutic agents can be one or more antioxidants.

Certain antioxidants contemplated for co-administration with compounds of Formula (I), or pharmaceutically acceptable salts thereof, include the following: vitamins, such as vitamin C and vitamin E; glutathione, lipoic acid, uric acid, sulforaphane carotenes, lycopene, lutein, anthocyanins, oxalic acid, phytic acid, tannins, coenzyme Q, melatonin, tocopherols, tocotrienols, polyphenols including resveratrol, flavonoids, selenium, eugenol, idebenone, mitoquinone, mitoquinol, ubiquinone, Szeto-Schiller peptides, and mitochondrial-targeted antioxidants.

Additional antioxidants contemplated for co-administration with compounds of Formula (I), or pharmaceutically acceptable salts thereof, include those compounds disclosed in U.S. Patent Nos. 6,331,532; 7,179,928; 7,232,809; 7,888,334; 7,888,335; 7,432,305; 7,470,798; and 7,514,461; and U.S. Patent Application Nos. 20020052342; 20030069208; 20040106579; 20050043553; 20050245487; 20060229278; 20070238709; 20070270381; 20080161267; 20080275005; 20090258841; 20100029706; and 20110046219;

In some embodiments, compounds of Formula (I), compounds of Formula (II), or pharmaceutically acceptable salts of any of the foregoing, may be co-administered with one or more cholinergic agonists; anticholinesterase agents; muscarinic antagonists; sympathomimetic agents; α- and β-andrenergic antagonists; carbonic anhydrase inhibitors; prostaglandin analogs; and marijuana, or extracts thereof.

### Liposomes and Liposomal Compositions

Some embodiments provide a liposomal composition comprising from about 1% to about 99% of one or more deuterated compounds in the liposome bilayer, where the deuterated compounds comprises a deuterated polyunsaturated fatty acid or analogs thereof appended to a phospholipid (including but not limited to phosphatidylethanolamines, phosphatidylcholines, phosphatidylserines, phosphoinositides, as well as non-glyceryl based phosphosphingolipids, such as ceramides and sphingomyelins). In some embodiments, the deuterated compound has the structure of Formula (I) (including formulas (IA), (IB), (IC) and (ID)) or Formula (II) (including formulas (IIA), (IIB), (IIC) and (IID)), where R¹ or R^{1A} is a glyceryl ester described herein or sphingolipid. The glyceryl ester may be in the form of a phosphatidylethanolamine, a phosphatidylcholine, a phosphatidylserine, or a phosphoinositide. In some embodiments, one or more deuterated compounds in the lipid bilayer may include the following structures: 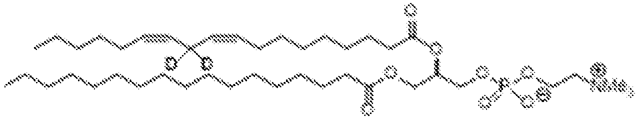 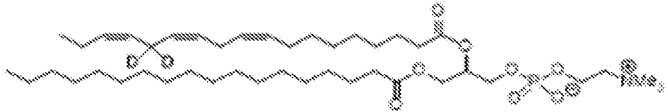 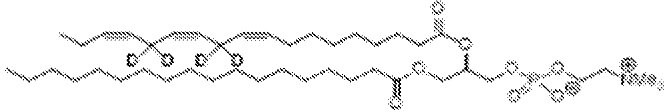 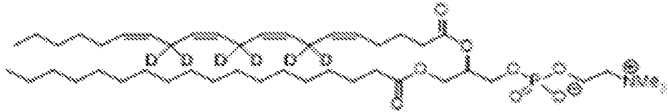 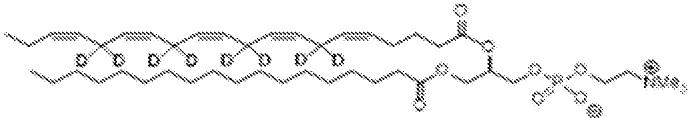 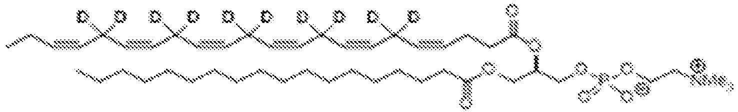 or a salt of any of the foregoing. In other embodiments, the deuterated compound in the liposome composition may be less than 1%.

In some embodiments, the liposome bilayer further comprises non-deuterated lipids, for example, one or more of linoleic acid, linolenic acid, arachidonic acid, docosahexaenoic acid, eicosapentaenoic acid, phosphatidylcholine, phosphatidylethanolamine, phosphatidylserine, phosphatidylinositol, sphingomyelin, lysophosphatidylcholine, and phosphatidylglycerine.

In some embodiments, the bilayer of the liposomes comprise about 1% to about 99% of deuterated compounds, or salts thereof. In some instances, the bilayer of the liposomes comprise about 1% to about 50% of deuterated compounds, or salts thereof. In other instances, the bilayer of the liposomes comprise about 1% to about 20% of deuterated compounds, or salts thereof. In still other instances, the bilayer of the liposomes comprise about 1% to about 5% of deuterated compounds, or salts thereof. In some instances, the bilayer of the liposomes comprise about 1% of deuterated compounds, or salts thereof. In some other instances, the bilayer of the liposomes may comprise less than about 1% of deuterated compounds.

In some embodiments, the liposomes are resistant to autooxidation relative to an identical liposomal composition with undeuterated compounds. Measuring lipid oxidation in a bilayer can be conducted using standard experimental techniques, for example, by a trapped fluorophore leakage, BODIPY assay, or conjugated diene absorbance (A₂₃₄) measurement. The liposomal compositions described herein may be used both as in vitro model systems, as well as for the delivery of oxidation-sensitive substances, such as H-PUFAs or other therapeutic agents, to a subject in need thereof.

### EXAMPLES

The exemplified compounds which are not encompassed by the scope of the claims do not form part of the present invention.

### Example 1.

In this example, the effect of various deuterated polyunsaturated fatty acids (D-PUFAs) on the stability of liposomes under oxidative stress conditions were investigated. A relatively small fraction of D-PUFAs was introduced into cells. The permeability of vesicle membranes to fluorescent dyes was measured as a proxy for bilayer integrity, and the formation of conjugated dienes was monitored as a proxy for lipid peroxidation (LPO).

**Chemical** synthesis. Soybean lysophosphatidylcholine (lysolipid, Lipoid) and other commercial reagents and solvents were used as received. Chloroform was washed with water, dried with CaCl₂ and distilled over P₂O₅. ¹H (500 MHz) and ¹³C (126 MHz) NMR spectra were obtained with Bruker DRX-500 spectrometer and referenced to residual solvent signals (CDCl₃: 7.26 ppm for ¹H; CD₃OD: 4.87 ppm for 1H and 49.00 ppm for ¹³C). Analytical thin-layer chromatography was performed on TLC Silica gel 60 F₂₅₄ (Merck). Silica gel column chromatography was performed using Merck Kieselgel 60 0.063-0.200 nm. Scheme A depicts the generic synthetic route for certain deuterated PUFAs described herein. Scheme B depicts a generic synthetic route for the phosphoamino groups used herein. 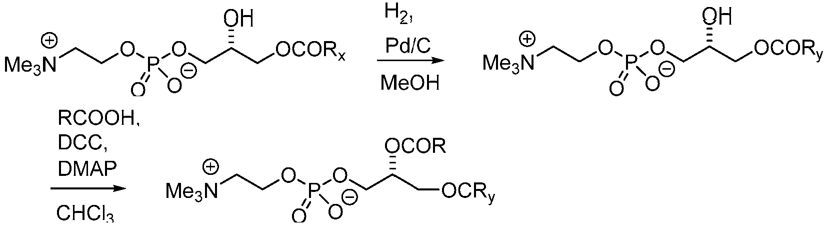 Rₓ = -alkyl, -alkenyl, or -alkynyl; Ry = -alkyl; R= Formula (II) moiety without -C(O)-R^{1A}

**Hydrogenated lysophosphatidylcholine (H-LPC, 2).** Soybean lysophosphatidylcholine (3.5 g) was dissolved in MeOH (50 mL) and 5% Pd/C (350 mg) was added to the solution. The mixture was stirred under H₂ (1 atm) for 1 h, filtered through a silica plug and cooled to 0 °C overnight. The resulting crystalline product was filtered off, washed with cold MeOH and dried *in vacuo.* The filtrate was evaporated to give another portion of product. Yield 3.39 g (95%). ¹H NMR (CD₃OD) δ 4.28 (m, 2H), 4.17 (dd, *J =* 11.3, 4.6 Hz, 2H), 4.10 (dd, *J =* 11.3, 6.4 Hz, 2H), 4.00-3.85 (m, 3H), 3,64 (m, 2H), 3.34 (s, 2H), 3.30 (p, *J =* 1.7 Hz, 3H), 3.22 (s, 9H), 2.35 (t, *J =* 7.5 Hz, 2H), 1.61 (p, *J =* 7.2 Hz, 2H), 1.28 (m, 28H), 0.89 (t, *J =* 6.9 Hz, 3H). ¹³C NMR (CD₃OD) δ 175.34, 69.79, 67.84, 67.47, 66.22, 60.45, 54.66, 34.91, 33.09, 30.79, 30.64, 30.49, 30.45, 30.25, 26.00, 23.75, 14.46.

This was followed by a coupling of a free D-PUFA to the SN2 position of lysolipid: 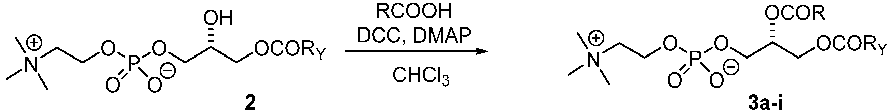 **3a**: RCOOH = linoleic acid; **3b**: RCOOH = 11,11-D₂-linoleic acid; **3c**: RCOOH = arachidonic acid; **3d**: RCOOH = 7,7,10,10,13,13-D₆-arachidonic acid; **3e**: RCOOH = linolenic acid; **3f**: RCOOH = 11,11,14,14-D₄-linolenic acid; **3h:** RCOOH = 7,7,10,10,13,13,16,16-D₈-eicosapentaenoic acid; 3i: RCOOH = 6,6,9,9,12,12,15,15,18,18-D₁₀-docosahexaenoic acid

**Acylated H-LPCs (3a-i).** Intermediate **2** (300 mg, 0.57 mmol) was dissolved in CHCl₃ (10 mL), then fatty acid (1.15 mmol), 4-DMAP (140 mg, 1.15 mmol) and DCC (236 mg, 1.15 mmol) were added. The reaction mixture was stirred at 20 °C for 5 days, the solids were filtered and washed with CHCl₃. The solvent was evaporated and the residue was chromatographed on silica in CHCl₃, CHCl₃ - MeOH (2:1, 1:1, 1:2) and MeOH. The pure fractions were evaporated to give product as a colorless waxy substance. *R_{f}* 0.40 (CHCl₃, MeOH, conc. NH₃ 13:5:1).

**1-Acyl-2-linoleyl-*sn*-glycero-3-phosphatidylcholine (3a).** Yield 344 mg (77%). ¹H NMR (CDCl₃) δ 5.36 (m, 4H), 4.41 (m, 1H), 4.30 (m, 2H), 4.11 (dd, *J =* 12.1, 7.4 Hz, 1H), 4.00-3.85 (m, 2H), 3,76 (m, 2H), 3.33 (m, 9H), 2.80 (m, 2H), 2.28 (m, 4H), 2.10 (m, 4H), 1.58 (m, 4H), 1.24 (m, 40H), 0.97 (m, 6H).

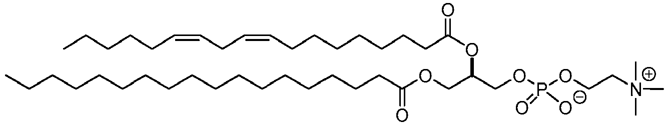

**1-Acyl-2-(11,11-D₂-linoleyl)-*sn*-glycero-3-phosphatidylcholine (3b).** Yield 364 mg (81%). ¹H NMR (CDCl₃) δ 5.36 (m, 4H), 4.41 (m, 1H), 4.30 (m, 2H), 4.11 (dd, *J* = 12.1, 7.4 Hz, 1H), 4.00-3.85 (m, 2H), 3,76 (m, 2H), 3.33 (m, 9H), 2.28 (m, 4H), 2.10 (m, 4H), 1.58 (m, 4H), 1.24 (m, 40H), 0.97 (m, 6H).

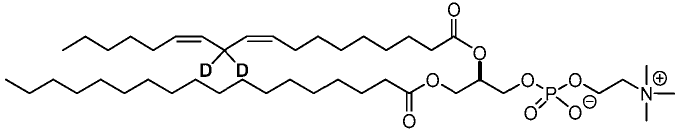

**1-Acyl-2-arachidonoyl*-sn*-glycero-3-phosphatidylcholine (3c).** Yield 381 mg (82%). ¹H NMR (CDCl₃) δ 5.36 (m, 8H), 5.19 (m, 1H), 4.41 (m, 1H), 4.30 (m, 2H), 4.11 (dd, *J=* 12.1, 7.4 Hz, 1H), 4.00-3.85 (m, 2H), 3,76 (m, 2H), 3.33 (m, 9H), 2.80 (m, 6H), 2.28 (m, 4H), 2.10 (m, 4H), 1.58 (m, 4H), 1.24 (m, 38H), 0.97 (m, 6H).

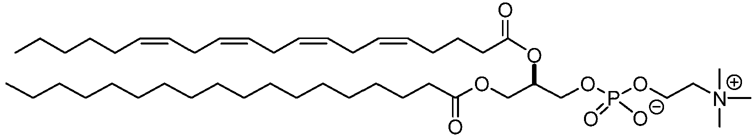

**1-Acyl-2-(7,7,10,10,13,13-D₆-arachidonoyl)-*sn*-glycero-3-phosphatidylcholine (3d).** Yield 350 mg (75%). ¹H NMR (CDCl₃) δ 5.36 (m, 8H), 5.19 (m, 1H), 4.41 (m, 1H), 4.30 (m, 2H), 4.11 (dd, *J =* 12.1, 7.4 Hz, 1H), 4.00-3.85 (m, 2H), 3,76 (m, 2H), 3.33 (m, 9H), 2.28 (m, 4H), 2.10 (m, 4H), 1.58 (m, 4H), 1.24 (m, 38H), 0.97 (m, 6H).

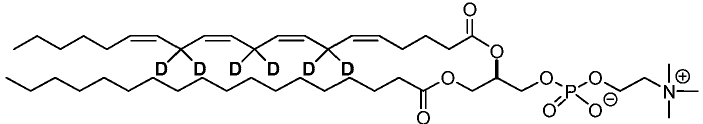

**1-Acyl-2-linolenyl-*sn-*glycero-3-phosphatidylcholine (3e).** Yield 330 mg (74%). ¹H NMR (CDCl₃) δ 5.34 (m, 6H), 5.19 (m, 1H), 4.38 (m, 1H), 4.28 (m, 2H), 4.11 (dd, *J =* 12.1, 7.4 Hz, 1H), 4.00-3.85 (m, 2H), 3,76 (m, 2H), 3.33 (m, 9H), 2.80 (m, 4H), 2.28 (m, 4H), 2.10 (m, 4H), 1.58 (m, 4H), 1.24 (m, 38H), 0.97 (t, *J=* 7.5 Hz, 3H), 0.89 (t, *J =* 6.9 Hz, 3H).

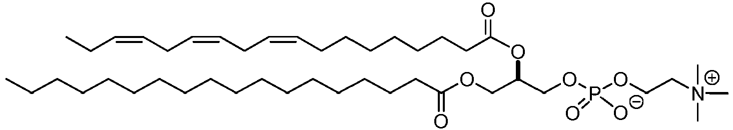

**1-Acyl-2-(11,11,14,14-D₄-linolenyl)-*sn*-glycero-3-phosphatidylcholine (3f).** Yield 410 mg (90%). ¹H NMR (CDCl₃) δ 5.34 (m, 6H), 5.19 (m, 1H), 4.38 (m, 1H), 4.28 (m, 2H), 4.11 (dd, *J =* 12.1, 7.4 Hz, 1H), 4.00-3.85 (m, 2H), 3,76 (m, 2H), 3.33 (m, 9H), 2.28 (m, 4H), 2.05 (m, 4H), 1.58 (m, 4H), 1.24 (m, 38H), 0.97 (t, *J =* 7.5 Hz, 3H), 0.89 (t, *J =* 6.9 Hz, 3H).

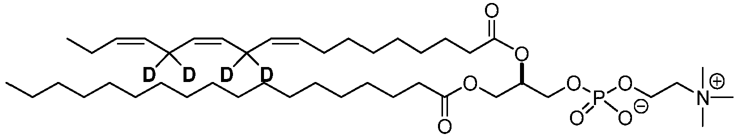

**1-Acyl-2-(14,14-D₂-linolenyl)-*sn*-glycero-3-phosphatidylcholine (3g).** Yield 366 mg (81%). ¹H NMR (CDCl₃) δ 5.34 (m, 6H), 5.19 (m, 1H), 4.38 (m, 1H), 4.28 (m, 2H), 4.11 (dd, *J =* 12.1, 7.4 Hz, 1H), 4.00-3.85 (m, 2H), 3,76 (m, 2H), 3.39 (m, 9H), 2.80 (m, 2H), 2.28 (m, 4H), 2.10 (m, 4H), 1.58 (m, 4H), 1.24 (m, 38H), 0.97 (t, *J =* 7.5 Hz, 3H), 0.89 (t, *J =* 6.9 Hz, 3H).

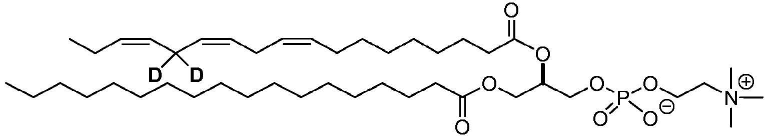

**1-Acyl-2-(7,7,10,10,13,13,16,16-D₈-eicosapentaenoyl)-*sn*-glycero-3-phosphatidylcholine (3h).** Yield 371 mg (80%). ¹H NMR (CDCl₃) δ 5.36 (m, 10H), 5.19 (m, 1H), 4.39 (dd, *J =* 12.0, 2.9 Hz, 1H), 4.28 (m, 2H), 4.11 (dd, *J =* 12.1, 7.3 Hz, 1H), 4.00-3.85 (m, 2H), 3,76 (m, 2H), 3.33 (m, 9H), 2.31 (t, *J =* 7.6 Hz, 2H), 2.26 (t, *J =* 7.6 Hz, 2H), 2.10 (m, 2H), 1.66 (m, 2H), 1.58 (m, 2H), 1.24 (m, 28H), 0.97 (t, *J =* 7.5 Hz, 3H), 0.89 (t, *J =* 6.9 Hz, 3H).

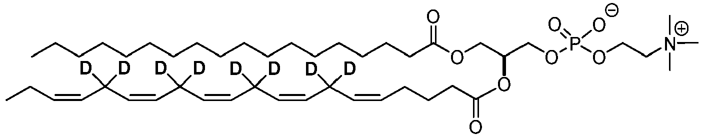

**1-Acyl-2-(6,6,9,9,12,12,15,15,18,18-D₁₀-docosahexaenoyl)-*sn*-glycero-3-phosphatidylcholine (3i).** Yield 390 mg (81%). ¹H NMR (CDCl₃) δ 5.36 (m, 12H), 5.19 (m, 1H), 4.38 (m, 1H), 4.28 (m, 2H), 4.12 (dd, *J =* 12.1, 7.4 Hz, 1H), 4.00-3.85 (m, 2H), 3,76 (m, 2H), 3.67 (m, 4H), 3.33 (m, 9H), 2.36 (m, 4H), 2.28 (m, 2H), 1.58 (m, 2H), 1.24 (m, 28H), 0.97 (t, *J =* 7.5 Hz, 3H), 0.89 (t*, J =* 6.9 Hz, 3H).

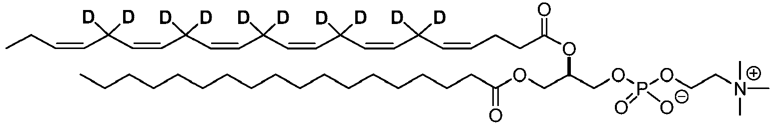

### Liposome preparation

Dye-loaded liposomes were prepared by evaporation under a stream of nitrogen of a 2% solution of a mixture of lipids in chloroform followed by hydration with a buffer solution containing fluorescent marker. To 5 mg of appropriate phosphatidylcholine mixture, 0.5 ml of 1 mM SRB in 100 mM KCl, 10 mM Tris, 10 mM MES, pH 7.4 was added. The mixture was vortexed, passed through several cycles of freezing and thawing, and extruded through 0.1-µm pore size Nucleopore polycarbonate membranes using an Avanti Mini-Extruder. The unbound marker was then removed by passage through a Sephadex G-50 coarse column (for SRB) with a buffer solution containing 100 mM KCl, 10 mM Tris, 10 mM MES, pH 7.4.

### Fluorescence correlation spectroscopy

Deuterated PUFAs, such as compounds of Formula (I), compounds of Formula (II), or pharmaceutically acceptable salts of any of the foregoing, when incorporated into lipid bilayers of liposomes, increase the resistance of liposomes to oxidative stress and lipid autooxidation in the non-linear fashion. Liposome leakage method is a convenient way to monitor lipid bilayer integrity. However, a relatively large size of sulforhodamine B (SRB) may require a rather extensive degree of membrane destruction for the dye to be able to leak out, which may be too substantial compared to processes typically damaging membranes *in vivo,* even at pathological conditions. Therefore, the conjugated diene method was also used to cover a wider dynamic range of lipid autooxidation.

The equipment set up and peak intensity analysis was conducted as described in Perevoshchikova, et al., Biochim. Biophys. Acta 1778, 2182-2190 (2008). Briefly, fluorescence excitation and detection utilized a Nd:YAG solid state laser with a 532-nm beam attached to an Olympus IMT-2 epifluorescent inverted microscope equipped with a 40x, NA 1.2 water immersion objective (Carl Zeiss, Jena, Germany). The fluorescence light passed through an appropriate dichroic beam splitter and a long-pass filter and was imaged onto a 50-µm core fiber coupled to an avalanche photodiode (SPCM-AQR-13-FC, PerkinElmer Optoelectronics, Vaudreuil, Quebec, Canada). The signal from an output was sent to a PC using a fast interface card (Flex02-01D/C, Correlator.com, Bridgewater, NJ). The data acquisition time was 30 s. The fluorescence was recorded from the confocal volume located at about 50 µm above the coverslip surface with 50 µL of the buffer solution added. Most of the data were collected under the conditions of stirring a suspension by a paddle-shaped 3-mm plastic bar rotated at 600 rpm. To calibrate the setup, the fluorescence autocorrelation function of Rhodamine 6G solution was recorded. Assuming the diffusion coefficient of the dye to be 2.5 x 10⁻⁶ cm²/s, the value of the confocal radius is circa ω=0.42 µm. The correlated fluorescence emission signals were fitted to the three-dimensional autocorrelation function as described in Hess, et al., Biochemistry 41; 697-705 (2002).

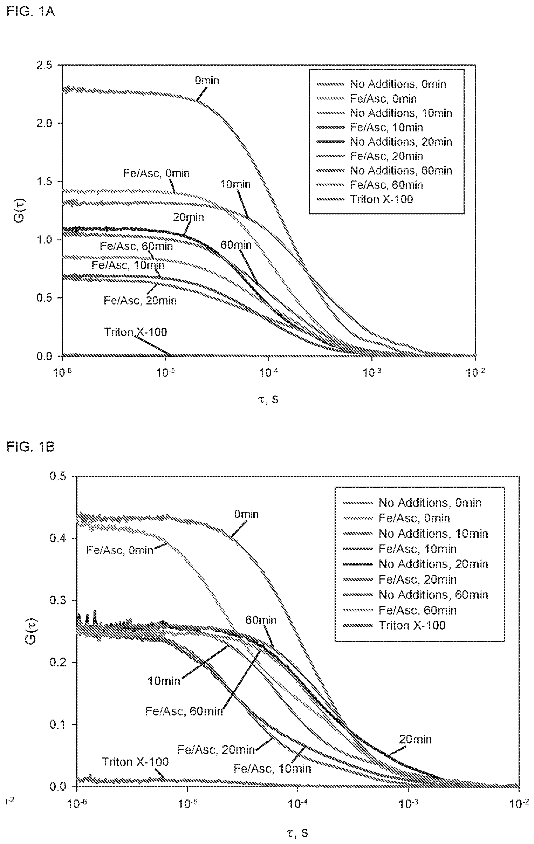

SRB-loaded liposomes were suspended in an aqueous solution of Fe²⁺ and ascorbate on G(τ), prepared from lipid mixtures containing linoleoyl stearoyl phosphatidylcholine (H-Lin-PC) as the bulk lipid (unless otherwise stated) with various contents of a series of 1-stearoyl phosphatidylcholines, including compounds of Formula (II). To measure G(τ→0) more precisely, the FCS experiments were performed under stirring conditions. FIG. 1A shows a timedependence of the G(τ) functions of SRB for liposomes prepared from H-Lin-PC lipids with 10% D₂-Lin-PC measured without Fe²⁺/ascorbate at 0 minute, 10 minute and 20 minutes and after the addition of Fe/ascorbate at 0 minute, 10 minute and 20 minute. The incubation with Fe/ascorbate led to a decrease in the G(τ→0) amplitude. Disruption of liposomes by the addition of Triton X-100 resulted in a drop of G(τ→0) to nearly zero bottom line in FIG.1A . FIG.1B shows the same type of experiment carried out with liposomes having 25% D₂-Lin-PC in H-Lin-PC. In this case, the incubation with Fe²⁺/ascorbate did not reduce the amplitude of G(τ→0) or reduced it to a very low extent. The reduction of the G(τ→0) amplitude apparently reflected an increase in the number of fluorescent particles due to SRB release from liposomes. Noteworthy, the contribution of different fluorescent species (here dye-loaded liposomes and free dye molecules released from liposomes) to G(τ→0) is proportional to square of their brightness.

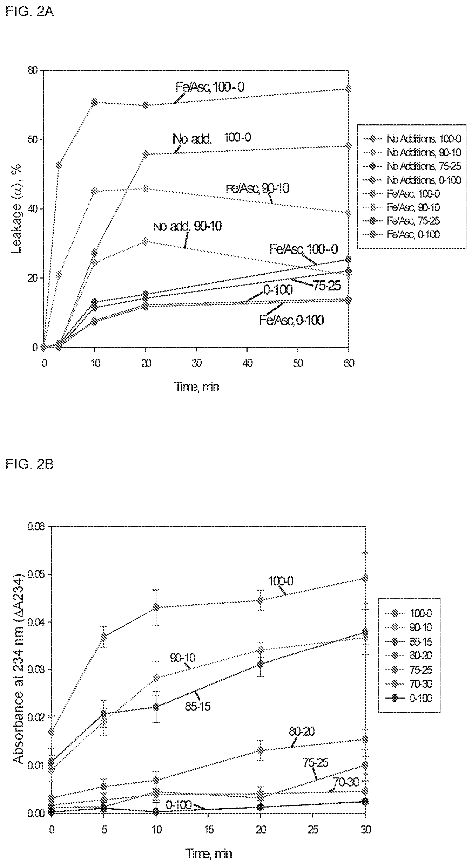

FIG. 2A displays time courses of the extent of liposome leakage α (plus/minus Fe²⁺/ascorbate) for liposomes having different% of D₂-Lin-PC (namely, 0%, 10%, 25%, and 100%). The difference between α with and without Fe²⁺/ascorbate was high for the 0% D₂-Lin-PC but was very low for 100% of the deuterated lipid.

### Accumulation of Conjugated Dienes

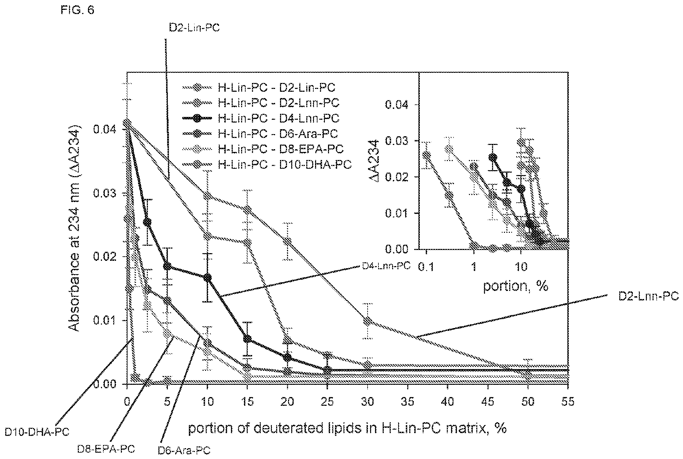

The accumulation of diene conjugates at various contents of D₁₀-DHA-PC in the undeuterated H-Lin-PC matrix were measured, compared to that for D₈-EPA-PC, D₆-Ara-PC, D₄-Lnn-PC, D₂-Lnn-PC and D₂-Lin-PC (FIG. 6). D₁₀-DHA-PC exhibited substantially enhanced protection from oxidative stress compared to D₆-Ara-PC and other deuterated lipids. The IC₅₀ concentration was about 0.2% for D₁₀-DHA-PC, while IC₅₀ for D₈-EPA-PC was about 1.5%, about 2% for D₆-Ara-PC, about 5% for D₄-Lnn-PC, about 22% for D₂-Lnn-PC, and about 15% for D₂-Lin-PC. The structures of these D-PUFA modified PC are shown below:

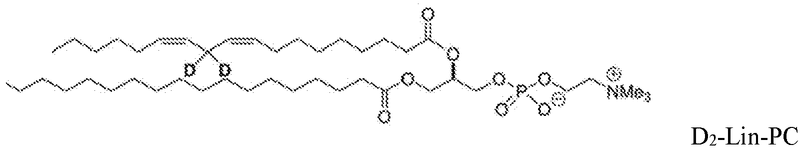

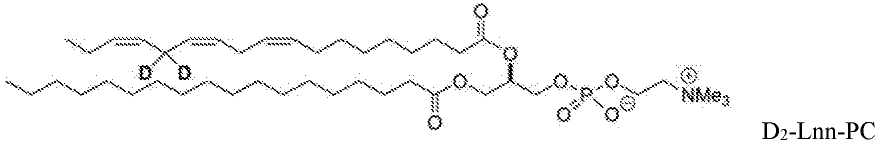

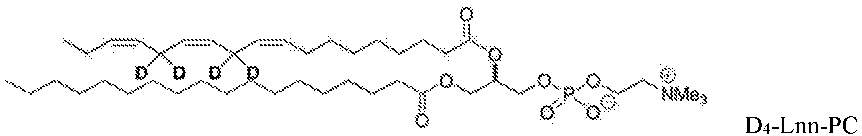

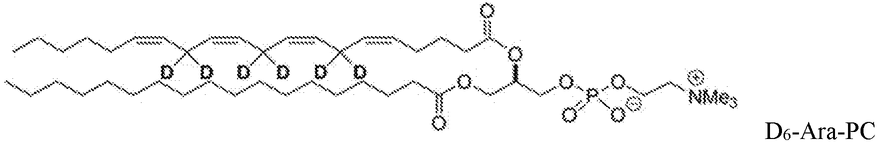

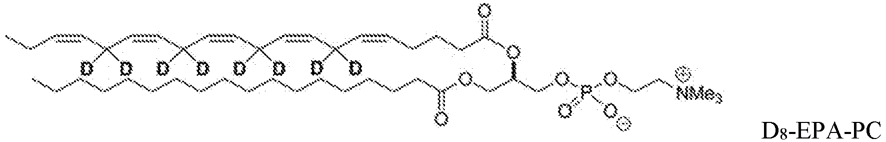

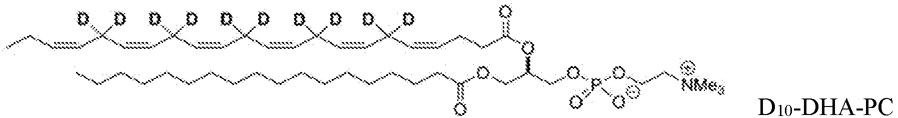

To compare the liposomes leakage induced by Fe²⁺/ascorbate with the process of lipid autooxidation, a conjugated diene assay was used, based on the measurement of absorption at 234 nm (A234). FIG. 2B shows the time courses of ΔA234 after the addition of Fe²⁺/ascorbate to liposomes having various % of D₂-Lin-PC. This parameter increased considerably for lipid mixtures with low percentage of deuterated D₂-Lin-PC and did not increase at high content of D₂-Lin-PC (FIG. 2B). However, A234 values were stable and did not increase in time without the addition of Fe²⁺/ascorbate.

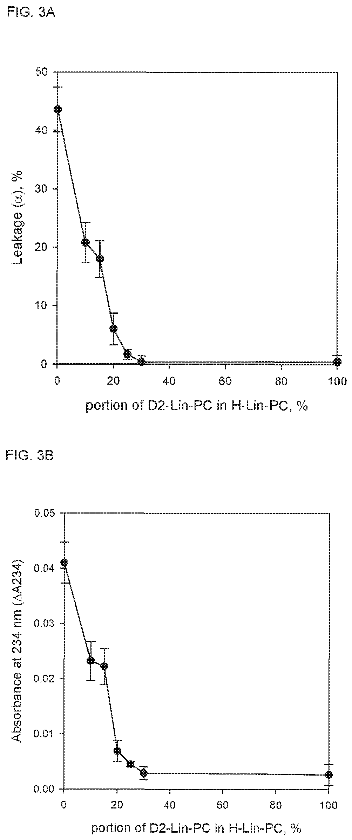

The dependences of ΔA234 at t=10 min (FIG. 3B) and of the difference in α +/- Fe²⁺/ascorbate (FIG. 3A) on the % of D₂-Lin-PC are shown. After a sharp decrease at low % of D₂-Lin-PC, the curves are close to each other, leading to a very low level of ΔA234 and a very low value of the difference in α at about 20% of D₂-Lin-PC and higher.

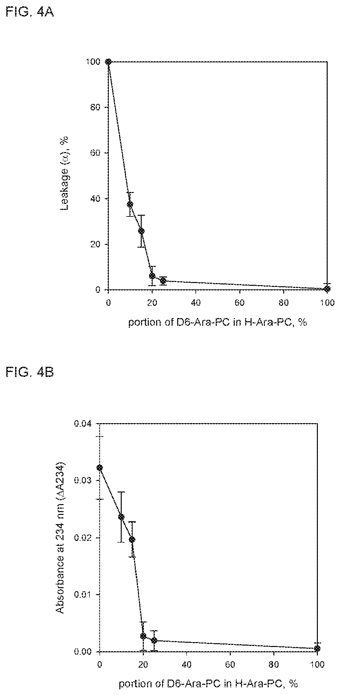

FIG. 4 shows the results of similar experiments with liposomes made from lipids containing arachidonic acid (H-Ara-PC) having various contents of D₆-Ara-PC. The protective effects of D₂-Lin-PC (FIG. 3) and D₆-Ara-PC (FIG. 4) exerted on the corresponding H-Lin-PC and H-Ara-PC were observed in a similar concentration range of about 10% to about 20% of the deuterated lipids.

The extinction coefficient ε for conjugated dienes has been estimated to be 28000 mol⁻¹ x cm⁻¹ at 234 nm. Therefore, for the maximal A234 measured at 0.05 (at 30 mins, FIG. 2B), the concentration of dienes is around 2 µM. For 10 µg/mL (or 10 µM) total lipids used, the level of conjugated dienes is about 20%.

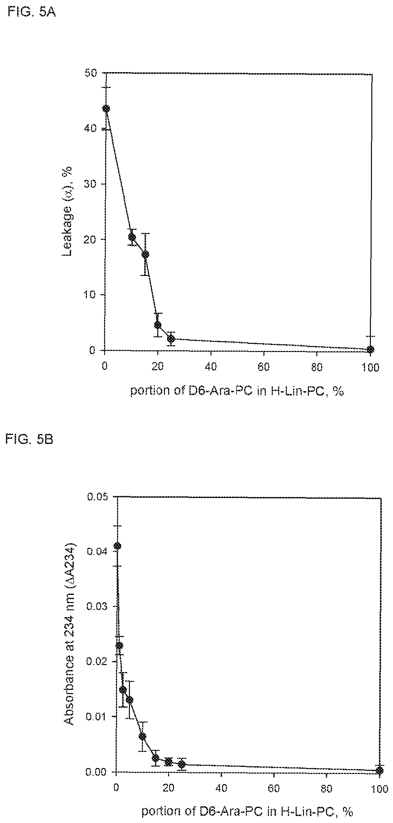

Comparison of the protecting effect of D₂-Lin-PC in the H-Lin-PC matrix with that of D₆-Ara-PC in the H-Lin-PC matrix reveals (FIG. 5) that the protective effect of D₆-Ara-PC on the H-Lin-PC liposome leakage required the same concentration of D₆-Ara-PC as in FIG. 4 (about 10% to about 20%), while the effect on diene conjugates required substantially smaller amounts of D₆-Ara-PC (about 5% to about 10%).

The accumulation of diene conjugates at various contents of D₁₀-DHA-PC in the undeuterated H-Lin-PC matrix was also measured, compared to that for D₈-EPA-PC, D₆-Ara-PC, D₄-Lnn-PC, D₂-Lnn-PC and D₂-Lin-PC (FIG. 6). Surprisingly, D₁₀-DHA-PC exhibited substantially enhanced protection from oxidative stress compared to D₆-Ara-PC and other deuterated lipids. The IC₅₀ concentration was about 0.2% for D₁₀-DHA-PC, while IC₅₀ for D₈-EPA-PC was about 1.5%, about 2% for D₆-Ara-PC, about 5% for D₄-Lnn-PC, about 22% for D₂-Lnn-PC, and about 15% for D₂-Lin-PC.

1-stearoyl-phosphatidylcholines containing 11,11-D₂-linoleic (D₂-Lin-PC), 11,11,14,14-D₄-linolenic (D₄-Lnn-PC), 14,14-D₂-linolenic (D₂-Lnn-PC), 7,7,10,10,13,13-D₆-arachidonic (D₆-Ara-PC), 7,7,10,10,13,13,16,16-D₈-eicosapentaenoic (D₈-EPA-PC) and 6,6,9,9,12,12,15,15,18,18-D₁₀-docosahexaenoic (D₁₀-DHA-PC) acids at position 2 of glycerol group were prepared as described herein. The formation of liposomes and their subsequent processing are typically performed in air. To minimize the non-specific oxidation of H-PUFA-PCs during liposome preparation, H-Lin-PC was used as a non-deuterated bulk component of bilayers, except in FIG. 4, where H-Ara-PC was used. It was discovered that the relative strength of protection of H-Lin-PC bulk by various D-PUFA-PCs increased with increasing number of deuterated bis-allylic CD₂ groups in a D-PUFA. For example, the D₂-Lin-PC (at 25% in bilayer), D₂-Lnn-PC, D₄-Lnn-PC (17%), D₆-Ara-PC (12%) and Ds-EPA-PC (10%) series (FIG. 6). D₁₀-DHA-PC also followed this trend. Surprisingly, the protection rendered by D₁₀-DHA-PC, suppressing lipid autooxidation at levels as low as 1% in the H-Lin-PC matrix, as measured by the diene conjugates assay (FIG. 6), was substantially stronger (e.g., 10-fold stronger than for D₆-Ara) than would be expected simply from the total number of its -CD₂- groups.

It was also discovered that the percentage of D₆-Ara-PC, needed for protection of H-Ara-PC matrix, is similar to that found for the case of D₂-Lin-PC in the H-Lin-PC matrix (FIG.6). Besides, the percentage of D₂-Lnn-PC needed for protection of H-Lin-PC matrix is about twofold as high as that of D₄-Lnn-PC (see FIG. 6). Increasing the total number of such "-CD₂-equivalents" seems to "buffer" the chain propagating radicals, slowing them down thus inhibiting lipid autooxidation. Thus, compounds of Formula (I), compounds of Formula (II), and pharmaceutically acceptable salts of any of the foregoing, provide effective protection of membrane integrity (FIGs. 3 and 4) at levels as low as 1% in the bilayer.

### Example 2

In this example, D-DHA (20mg; 1mg/ul) was administered orally as ethyl ester to a wild-type mouse by gavage for three consecutive days in addition to unrestricted regular DHA-free rodent diet. Mouse retina was harvested and then fixed and embedded in resin. Retinal sections were analyzed with nanoscale secondary ion mass spectrometry (nanoSIMS) to determine the incorporation rates of D-DHA into retinal cells by measuring the relative enrichment of DHA-bound deuterium over its natural tissue abundance. The D-DHA/DHA substitution rate of natural DHA in the outer segments was calculated by the ratio of increased deuterium abundance vs. total hydrogen contribution of DHA in outer segments. The result showed that about 1.9% to 2.5% of DHA has been replaced with D-DHA. In conclusion, it was observed that orally dosed D-DHA is delivered to retinal tissues via the blood stream and reaches effective doses above 1% after three days of dosing.

## Claims

1. A compound of Formula (I), or a pharmaceutically acceptable salt thereof: 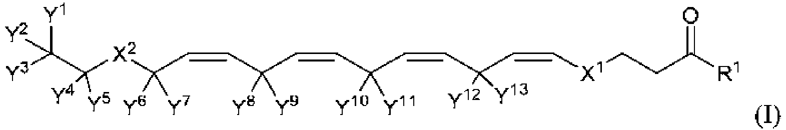 wherein:
each of Y¹, Y², Y³, Y⁴, Y⁵, Y⁶, Y⁷, Y⁸, Y⁹, Y¹⁰, Y¹¹, Y¹², Y¹³, Y¹⁴, and Y¹⁵ are independently hydrogen and deuterium;
X¹ is -C(Y¹⁴Y¹⁵)-CH=CH-, -CH₂-, -CD₂-, or a direct bond;
X² is -CH₂CH₂-, -CH₂CD₂-, -CD₂CH₂-, -CD₂CD₂-, or -CH=CH-;
R¹ is a substituted or unsubstituted -O-C₁-C₆ alkyl, a substituted or unsubstituted -S-C₁-C₆ alkyl, a substituted or unsubstituted -NH-C₁-C₆ alkyl, -NH₂, - OH, an unsubstituted sphingolipid, or an unsubstituted glyceryl ester;
wherein at least two of Y¹, Y², Y³, Y⁴, Y⁵, Y⁶, Y⁷, Y⁸, Y⁹, Y¹⁰, Y¹¹, Y¹², Y¹³ ,Y¹⁴, and Y¹⁵ are independently deuterium;
wherein when each of Y¹-Y⁵ are deuterium, then X¹ is -C(Y¹⁴Y¹⁵)-CH=CH- and at least one of Y⁶-Y¹⁵ is deuterium; or X¹ is -CD₂-; or X² is -CH₂CD₂-, -CD₂CH₂-, or - CD₂CD₂-;
wherein the compound of Formula (I), or a pharmaceutically acceptable salt thereof, comprises from about 1% to about 99% of the total amount of fats, fatty acids, and fatty acid esters administered to, or ingested by, the subject,
for use in treating or ameliorating a retinal disease or condition caused by lipid autooxidation in a subject in need thereof, wherein the retinal disease or condition is Leber's hereditary optic neuropathy (LHON), macular telangiectasia, or glaucoma.

2. The compound for use of Claim 1, wherein the compound of Formula (I), or a pharmaceutically acceptable salt thereof is incorporated into the subject's body following administration.

3. The compound for use of Claim 1 or Claim 2, wherein X¹ is -C(Y¹⁴Y¹⁵)-CH=CH- or -CH₂-.

4. The compound for use of any one of Claims 1 to 3, wherein X² is -CH=CH- or - CH₂CH₂-.

5. The compound for use of any one of Claims 1 to 4, wherein the compound of Formula (I) has the structure of Formula (IA), (IB), (IC), or (ID): 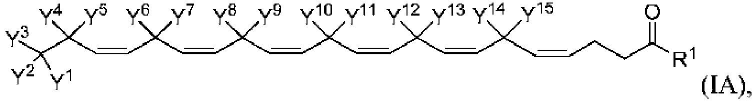 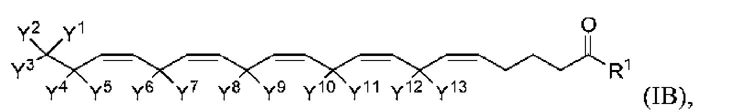 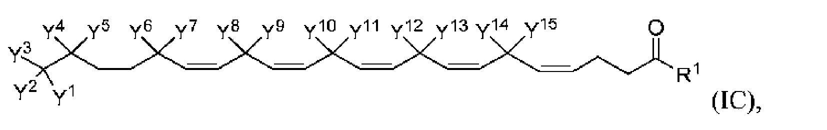 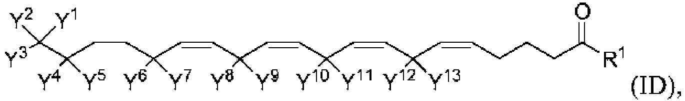 or a pharmaceutically acceptable salt of any of the foregoing.

6. The compound for use of any one of Claims 1 to 5, wherein R¹ is an unsubstituted -O-C₁-C₆ alkyl or -OH, such as wherein R¹ is -OCH₂CH₃.

7. The compound for use of any one of Claims 1 to 5, wherein R¹ is an unsubstituted glyceryl ester selected from the group consisting of an unsubstituted monoglyceryl ester; an unsubstituted diglyceryl ester; and an unsubstituted triglyceryl ester, such as wherein the unsubstituted glyceryl ester is acyl 1,3-dihydroxypropan-2-yl.

8. The compound for use of any one of Claims 1 to 7, wherein the compound of Formula (I), or a pharmaceutically acceptable salt thereof, comprises:
(a) from about 1% to about 10% of the total amount of fats, fatty acids, and fatty acid esters administered to, or ingested by, the subject; or
(b) less than, or about 5% of the total amount of fats, fatty acids, and fatty acid esters administered to, or ingested by, the subject, such as less than, or about 1% of the total amount of fats, fatty acids, and fatty acid esters administered to, or ingested by, the subject.

9. The compound for use of any one of Claims 1 to 8, wherein the compound of Formula (I) is not a deuterated arachidonic acid, or a salt or alkyl ester thereof.

## Patentansprüche

1. Verbindung der Formel (I) oder pharmazeutisch verträgliches Salz davon: 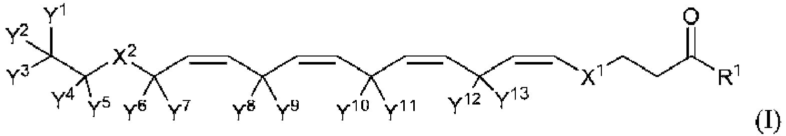 wobei:
jedes von Y¹, Y², Y³, Y⁴, Y⁵, Y⁶, Y⁷, Y⁸, Y⁹, Y¹⁰, Y¹¹, Y¹², Y¹³, Y¹⁴ und Y¹⁵ unabhängig voneinander Wasserstoff und Deuterium ist;
X¹ -C(Y¹⁴Y¹⁵) -CH=CH-, -CH₂-, -CD₂- oder eine direkte Bindung ist;
X² -CH₂CH₂-, -CH₂CD₂-, -CD₂CH₂-, -CD₂CD₂- oder -CH=CH- ist;
R¹ ein substituiertes oder unsubstituiertes -O-C₁-C₆-Alkyl, ein substituiertes oder unsubstituiertes -S-C₁-C₆-Alkyl, ein substituiertes oder unsubstituiertes -NH-C₁-C₆-Alkyl, -NH₂, -OH, ein unsubstituiertes Sphingolipid oder ein unsubstituierter Glycerylester ist;
wobei mindestens zwei von Y¹, Y², Y³, Y⁴, Y⁵, Y⁶, Y⁷, Y⁸, Y⁹, Y¹⁰, Y¹¹, Y¹², Y¹³, Y¹⁴ und Y¹⁵ unabhängig voneinander Deuterium sind;
wobei, wenn jedes von Y¹-Y⁵ Deuterium ist, X¹ -C(Y¹⁴Y¹⁵)-CH=CH-ist und mindestens eines von Y⁶-Y¹⁵ Deuterium ist; oder X¹ -CD₂-ist; oder X² -CH₂CD₂-, -CD₂CH2- oder -CD₂CD₂- ist;
wobei die Verbindung der Formel (I) oder ein pharmazeutisch verträgliches Salz davon von etwa 1 % bis etwa 99 % der Gesamtmenge an Fetten, Fettsäuren und Fettsäureestern umfasst, die dem Subjekt verabreicht oder von ihm eingenommen wird,
zur Verwendung bei der Behandlung oder Linderung einer Netzhautkrankheit oder eines Netzhautzustands, die/der durch Lipidautooxidation verursacht wird, bei einem Subjekt, das dessen bedarf, wobei es sich bei der Netzhautkrankheit oder dem Netzhautzustand um Lebersche hereditäre Optikusneuropathie (LHON), makuläre Teleangiektasie oder Glaukom handelt.

2. Verbindung zur Verwendung nach Anspruch 1, wobei die Verbindung der Formel (I) oder ein pharmazeutisch verträgliches Salz davon nach der Verabreichung in den Körper des Subjekts eingelagert ist.

3. Verbindung zur Verwendung nach Anspruch 1 oder Anspruch 2, wobei X¹ -C(Y¹⁴Y¹⁵) -CH=CH- oder -CH₂- ist.

4. Verbindung zur Verwendung nach einem der Ansprüche 1 bis 3, wobei X² -CH=CH- oder -CH₂CH₂- ist.

5. Verbindung zur Verwendung nach einem der Ansprüche 1 bis 4, wobei die Verbindung der Formel (I) die Struktur von Formel (IA), (IB), (IC) oder (ID): 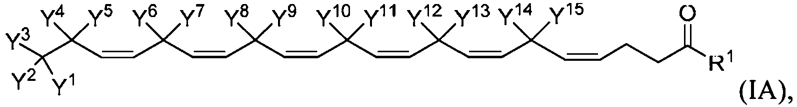 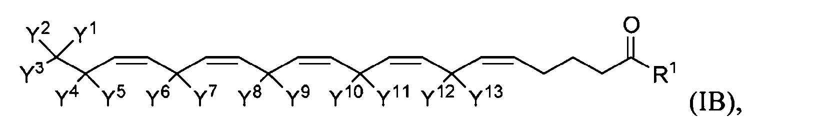 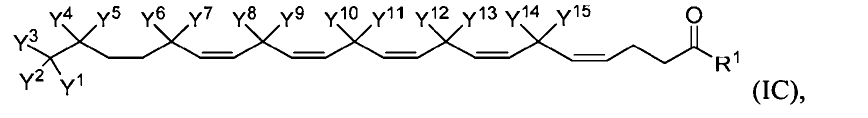 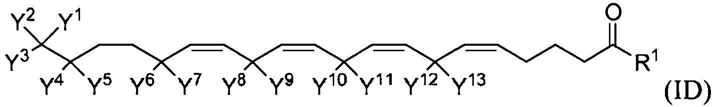 oder eines pharmazeutisch verträglichen Salzes einer der Vorstehenden aufweist.

6. Verbindung zur Verwendung nach einem der Ansprüche 1 bis 5, wobei R¹ ein unsubstituiertes -O-C₁-C₆-Alkyl oder -OH ist, wie wobei R¹ -OCH₂CH₃ ist.

7. Verbindung zur Verwendung nach einem der Ansprüche 1 bis 5, wobei R¹ ein unsubstituierter Glycerylester ist, ausgewählt aus der Gruppe, bestehend aus einem unsubstituierten Monoglycerylester; einem unsubstituierten Diglycerylester; und einem unsubstituierten Triglycerylester, wie wobei der unsubstituierte Glycerylester Acyl-1,3-dihydroxypropan-2-yl ist.

8. Verbindung zur Verwendung nach einem der Ansprüche 1 bis 7, wobei die Verbindung der Formel (I) oder ein pharmazeutisch verträgliches Salz davon Folgendes umfasst:
(a) von etwa 1 % bis etwa 10 % der Gesamtmenge an Fetten, Fettsäuren und Fettsäureestern, die dem Subjekt verabreicht oder von ihm eingenommen wird; oder
(b) weniger als oder etwa 5 % der Gesamtmenge an Fetten, Fettsäuren und Fettsäureestern, die dem Subjekt verabreicht oder von ihm eingenommen wird, wie weniger als oder etwa 1 % der Gesamtmenge an Fetten, Fettsäuren und Fettsäureestern, die dem Subjekt verabreicht oder von ihm eingenommen wird.

9. Verbindung zur Verwendung nach einem der Ansprüche 1 bis 8, wobei es sich bei der Verbindung der Formel (I) nicht um eine deuterierte Arachidonsäure oder ein Salz oder einen Alkylester davon handelt.

## Revendications

1. Composé de formule (I), ou un sel pharmaceutiquement acceptable de celui-ci : 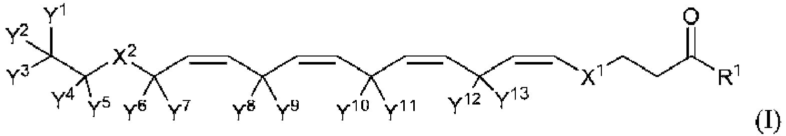 dans lequel :
chacun de Y¹, Y², Y³, Y⁴, Y⁵, Y⁶, Y⁷, Y⁸, Y⁹, Y¹⁰, Y¹¹, Y¹², Y¹³, Y¹⁴ et Y¹⁵ est indépendamment choisi parmi l'hydrogène et le deutérium ;
X¹ est -C(Y¹⁴Y¹⁵) -CH=CH-, -CH₂-, -CD₂-, ou une liaison directe ;
X² est -CH₂CH₂-, -CH₂CD₂-, -CD₂CH₂-, -CD₂CD₂-, ou -CH=CH- ;
R¹ est un groupe alkyle en C₁-C₆ substitué ou non substitué lié par -O-, un groupe alkyle en C₁-C₆ substitué ou non substitué lié par -S-, un groupe alkyle en C₁-C₆ substitué ou non substitué lié par -NH-, -NH₂, -OH, un sphingolipide non substitué ou un ester de glycéryle non substitué ;
dans lequel au moins deux parmi Y¹, Y², Y³, Y⁴, Y⁵, Y⁶, Y⁷, Y⁸, Y⁹, Y¹⁰, Y¹¹, Y¹², Y¹³, Y¹⁴, et Y¹⁵ sont indépendamment du deutérium ;
dans lequel lorsque chacun de Y¹ à Y⁵ est du deutérium, alors X¹ est -C(Y¹⁴Y¹⁵)-CH=CH- et au moins l'un de Y⁶ à Y¹⁵ est du deutérium ; ou X¹ est -CD₂- ; ou X² est -CH₂CD₂-, -CD₂CH2- ou - CD₂CD₂- ;
dans lequel le composé de formule (I), ou un sel pharmaceutiquement acceptable de celui-ci, comprend d'environ 1 % à environ 99 % de la quantité totale de graisses, d'acides gras et d'esters d'acides gras administrés au sujet ou ingérés par celui-ci,
destiné à être utilisé dans le traitement ou l'amélioration d'une maladie ou affection de la rétine causée par l'autooxydation des lipides chez un sujet qui en a besoin, dans lequel la maladie ou affection de la rétine est la neuropathie optique héréditaire de Leber (LHON), la télangiectasie maculaire ou le glaucome.

2. Composé destiné à être utilisé selon la revendication 1, dans lequel le composé de formule (I), ou un sel pharmaceutiquement acceptable de celui-ci, est incorporé dans l'organisme du sujet à la suite de son administration.

3. Composé destiné à être utilisé selon la revendication 1 ou la revendication 2, dans lequel X¹ est -C(Y¹⁴Y¹⁵) -CH=CH- ou -CH₂-.

4. Composé destiné à être utilisé selon l'une quelconque des revendications 1 à 3, dans lequel X² est -CH=CH- ou -CH₂CH₂-.

5. Composé destiné à être utilisé selon l'une quelconque des revendications 1 à 4, dans lequel le composé de formule (I) possède la structure de formule (IA), (IB), (IC) ou (ID) : 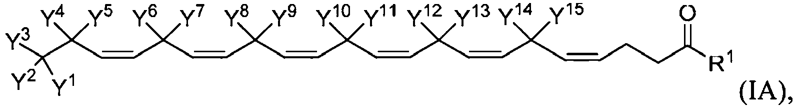 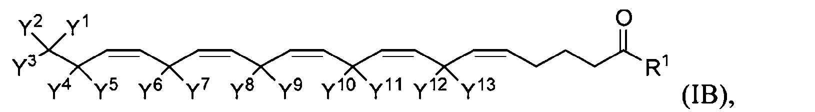 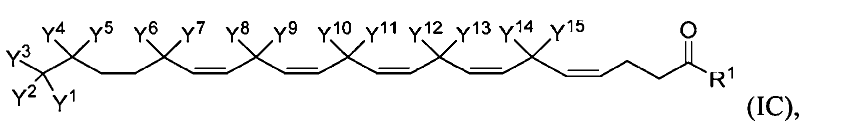 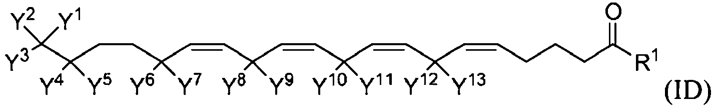 ou un sel pharmaceutiquement acceptable de l'un quelconque de ceux-ci.

6. Composé destiné à être utilisé selon l'une quelconque des revendications 1 à 5, dans lequel R¹ est un groupe alkyle en C₁-C₆ non substitué lié par -O- ou -OH, tel que R¹ est -OCH₂CH₃.

7. Composé destiné à être utilisé selon l'une quelconque des revendications 1 à 5, dans lequel R¹ est un ester de glycéryle non substitué choisi parmi dans le groupe consistant à un ester monoglycérylique non substitué, un ester diglycérylique non substitué et un ester triglycérylique non substitué, tel que l'ester de glycéryle non substitué est l'acyle 1,3-dihydroxypropan-2-yle.

8. Composé destiné à être utilisé selon l'une quelconque des revendications 1 à 7, dans lequel le composé de formule (I), ou un sel pharmaceutiquement acceptable de celui-ci, comprend :
(a) d'environ 1 % à environ 10 % de la quantité totale de graisses, d'acides gras et d'esters d'acides gras administrés au sujet ou ingérés par celui-ci ; ou
(b) moins de, ou environ 5 % de la quantité totale de graisses, d'acides gras et d'esters d'acides gras administrés au sujet ou ingérés par celui-ci, par exemple moins de, ou environ 1 % de la quantité totale de graisses, d'acides gras et d'esters d'acides gras administrés au sujet ou ingérés par celui-ci.

9. Composé destiné à être utilisé selon l'une quelconque des revendications 1 à 8, dans lequel le composé de formule (I) n'est pas un acide arachidonique deutéré, ni un sel ou un ester alkylique de celui-ci.
